# EUROPÄISCHE PATENTANMELDUNG

(11) **EP 4 306 135 A1**
(43) Veröffentlichungstag der Anmeldung: **17.01.2024**
(21) Anmeldenummer: 22184768.4
(22) Anmeldetag: 13.07.2022
(51) Int. Cl.: A61L 2/07, B01J 3/03, B01J 3/04, F16J 15/00

(54) **DAMPFSTERILISATOR**

(71) Anmelder: MELAG Medizintechnik GmbH & Co. KG, 10829 Berlin (DE)
(72) Erfinder: VON DER WAYDBRINK,Eberhard, 14641 Paulinenaue (DE); LITZBA, Guido, 14513 Teltow (DE); BECKER, Tino, 10117 Berlin (DE); LIEBETRAU, Matthias, 14612 Falkensee (DE); SCHMALZ, Elena, 10317 Berlin (DE); SCHULZ, Robert, 14552 Michendorf (DE); LICHT, Tobias, 13407 Berlin (DE); VON GRZYMALA, Benjamin, 16547 Birkenwerder (DE)
(74) Vertreter: Maikowski & Ninnemann Patentanwälte Partnerschaft mbB

(57) **Zusammenfassung**

Die Erfindung betrifft einen Dampfsterilisator, der einen Druckkessel (2) und ein mit dem Druckkessel (2) druckdicht verbundenes Mantelelement (3) aufweist, wobei zwischen dem Druckkessel (2) und dem Mantelelement (3) ein Dampfraum (4) ausgebildet ist. Erfindungsgemäß ist vorgesehen, dass der Druckkessel (2) und das Mantelelement (3) schweißfrei miteinander verbunden sind.

## Beschreibung

Die vorliegende Erfindung betrifft einen Dampfsterilisator gemäß dem Oberbegriff des Anspruchs 1 und ein Verfahren zur Herstellung eines solchen Dampfsterilisators gemäß dem Oberbegriff des Anspruchs 15.

Dampfsterilisatoren dienen der Sterilisation von Sterilisiergut mittels Dampf. Dabei wird das Sterilisiergut beispielsweise bei einer Temperatur von 121 °C oder 134 °C bei einem relativen Überdruck von etwa 1 oder 2 bar in einem Druckkessel des Dampfsterilisators behandelt, wobei auch niedrigere oder höhere Drücke grundsätzlich möglich sind. Während eines Sterilisationszyklus wird der Dampf in mindestens einem sogenannten Evakuierungsschritt aus dem Druckkessel mittels Unterdruck abgesaugt. Dabei werden Zieldrücke im Druckkessel im Bereich von etwa 80 mbar bis 500 mbar absolut erreicht. Mithin muss der Druckkessel so ausgelegt sein, dass er sowohl mit dem vorgenannten Überdrücken als auch mit den vorgenannten Unterdrücken problemlos beaufschlagt werden kann.

Der zur Sterilisation eingesetzte Dampf wird in einer Dampferzeugungseinrichtung hergestellt. Diese Dampferzeugungseinrichtung kann in dem Druckkessel des Dampfsterilisators angeordnet (interner Dampferzeuger) oder als externer Dampferzeuger ausgestaltet sein. Externe Dampferzeuger können in direkter, unterbrechungsfreier Strömungsverbindung mit dem Druckkessel stehen. Alternativ können sie auch mittels eines steuerbaren Ventils mit dem Druckkessel in Strömungsverbindung stehen (abgeriegelter Dampferzeuger, zentrale Dampfversorgung). Typischerweise wird sowohl die interne als auch die externe Dampferzeugung mittels eines elektrischen Heizkörpers im Dampferzeuger realisiert.

Besonders vorteilhaft sind externe Dampferzeuger in einem sogenannten Doppelmantel. Dabei ist ein Dampfraum in dem Doppelmantel ausgestaltet. Eine Manteloberfläche des Doppelmantels wird typischerweise durch eine Außenfläche des Druckkessels gebildet. Folglich kann der Doppelmantel dazu eingesetzt werden, den Druckkessel mittels einer einzigen Dampfquelle einerseits vorzuheizen und andererseits über ein steuerbares Ventil mit Dampf zu befüllen. Ferner ist es möglich, einen externen Dampferzeuger in Strömungsverbindung mit einem Doppelmantel zu bringen. Dann lässt sich der Doppelmantel aus dem externen Dampferzeuger mit Dampf versorgen, wobei der Dampf abermals zum Vorheizen des Druckkessels von außen dient. Der Doppelmantel fungiert dann auch als Dampf-Zwischenspeicher. Über ein steuerbares Ventil kann der Druckkessel bei Bedarf aus dem Zwischenspeicher, also aus dem Doppelmantel, mit Dampf versorgt werden.

Doppelmantel-Druckkessel werden üblicherweise mittels Schweißverfahren hergestellt, wobei es hierfür unterschiedliche Fertigungskonzepte gibt. Ein solches Schweißen ist jedoch ein recht aufwendiger Prozess, der hohe Anforderungen an die Fertigung und an die eingesetzten Materialien setzt. Demgegenüber können runde Druckkessel ohne Doppelmantel mittels einer Vielzahl von Herstellungsverfahren produziert werden. Beispielsweise können Sie komplett tiefgezogen werden, tiefgezogene Einzelteile aufweisen, die miteinander verschweißt werden, aus einem zum Rohr gewalzten Metallteil bestehen, dessen Längsnaht geschweißt wird und an das anschließend ein Klöpperboden angeschweißt wird. Grundsätzlich sind auch rechteckige Druckkessel herstellbar. Dies ist allerdings nur dann möglich, wenn sie stark abgerundete Ecken aufweisen. Typischerweise werden hierfür verschiedene miteinander verschweißte Einzelteile verwendet.

Auch die zum Betrieb eines Dampfsterilisators benötigten Anschlussstutzen (über die beispielsweise Dampf in den Druckkessel eingelassen oder aus dem Druckkessel evakuiert wird) werden üblicherweise direkt an den Druckkessel geschweißt. Dies kann entweder von innen oder von außen erfolgen.

Zur Sicherstellung einer hinreichenden Oberflächenqualität und einer dauerhaften Korrosionsbeständigkeit werden die Druckkessel von Dampfsterilisatoren im Rahmen der Herstellung elektropoliert, gebeizt, geschliffen und/oder gestrahlt. Schweißnähte müssen besonders sorgfältig ausgeführt werden, damit die Druckkessel die an sie gestellten Druckanforderungen erfüllen und über einen langen Zeitraum korrosionsbeständig sind.

Die DE 697 12 232 T2 beschreibt einen Doppelmantel-Druckkessel, der durch einen zylindrischen Mantel gebildet wird und Heizelemente in einem Zwischenraum zwischen dem äußeren und dem inneren Mantel aufweist. Dieser Zwischenraum muss dabei allerdings sehr groß ausgeführt werden, um eine ausreichende Wasseroberfläche für hohe Verdampfungsraten zu ermöglichen.

Eine ähnliche Lösung beschreibt die DE 699 03 151 T2, in der ein elliptischer Mantel um einen rechteckigen Druckkessel mit stark abgerundeten Ecken angeordnet ist. Durch die unterschiedlichen Formen des inneren und des äußeren Mantels werden unterschiedlich große Zwischenraumkompartimente gebildet, die zur Aufnahme von Wasser geeignet sind.

Die DE 102 60 895 A1 beschreibt einen Doppelmantel-Dampfsterilisator mit einem zylindrischen Dampfraum. An der Unterseite des Dampfraums ist ein Flüssigkeitsbecken zur Dampferzeugung angekoppelt. Dadurch lässt sich eine sehr gute Verdampfungsrate realisieren, wobei der Manteldurchmesser dennoch sehr gering sein kann, sodass der Platzbedarf des entsprechenden Dampfsterilisators ebenfalls gering ist. Allerdings ist die schweißtechnische Herstellung dieses Dampfsterilisators sehr aufwendig und entsprechend kostenintensiv.

Die EP 2 763 704 B1 beschreibt einen rechteckigen Druckkessel mit stark abgerundeten Ecken, der abermals von einem zylindrischen Mantel umgeben ist. Auch durch diese Ausgestaltung wird genügend Platz für die Integration von Heizkörpern in das Flüssigkeitsbecken bereitgestellt. Die Wasseroberfläche ist ebenfalls groß genug, um eine ausreichende Verdampfungsrate zu realisieren. Zudem entfallen Schweißarbeiten für das zusätzliche Flüssigkeitsbecken unterhalb des Doppelmantels, das bei der vorgenannten deutschen Patentanmeldung vorgesehen ist.

Die DE 10 2013 000 176 B4 beschreibt einen Doppelmantel-Dampfsterilisator, bei dem eine Vollmaterialronde an den Innenmantel und Außenmantel des Doppelmantels geschweißt ist. Dadurch kann das zusätzliche Anschweißen von Gewindebuchsen vermieden werden, die sonst zur Durchführung von Sensoren verwendet werden. Derartige Sensoren können stattdessen direkt in die Vollmaterialronde eingeschraubt werden. Die Sensoren können auf diese Weise genauer und tiefer platziert werden. Allerdings erfordert diese Lösung durch die Vollmaterialronde einen signifikant höheren Materialeinsatz als die anderen aus dem Stand der Technik bekannten Lösungen.

Das Patent US 8,206,660 B2 beschreibt einen Doppelmantel-Dampfsterilisator mit einer translatorisch verschiebbaren Tür. Dabei wird besonderes Augenmerk auf eine ausreichende Abdichtung dieser Tür gelegt.

Die EP 3 453 407 B1 beschreibt einen doppelwandigen drehbaren Behälter, der zur Sterilisation von saugfähigen Hygieneartikeln eingesetzt wird. Dabei wird ein Kondensatsammelbehälter mittels einer Schweißnaht an der Außenwand des Sterilisationsbehälters befestigt.

Die DE 10 2004 005 649 B3 beschreibt zwei miteinander verbundene Doppelmantel-Druckkessel, von denen der eine zur Sterilisation von Sterilisiergut eingesetzt wird und der andere als Referenzraum dient.

Die US 2006/0057021 A1 und die WO 1997/048420 A1 beschreiben jeweils Doppelmantel-Druckkessel, die - ähnlich wie eine Isolierkanne - ein konstantes Vakuum in ihrem Mantelraum aufweisen. Mithin würde Mantelraum hier nicht zur Erzeugung oder Durchleitung von Dampf eingesetzt, sondern isoliert aufgrund des in ihm herrschenden Vakuums.

Die EP 1 000 629 B1 und die EP 3 457 115 A1 beschreiben jeweils Doppelmantel-Druckkessel mit angeschlossener Sensorik bzw. verbundenen Messwertaufnehmern.

Die WO 2020/167428 A1, die WO 2021/231825 A2, die DE 10 2014 019 774 A1 und die EP 1 165 149 B1 beschreiben verschiedene Verfahren zum Betrieb von Doppelmantel-Druckkesseln. Dabei werden die Fertigungsverfahren, die zur Herstellung dieser Druckkessel angewandt werden, nicht näher erläutert. Anhand der Zeichnungen dieser Dokumente ist jedoch davon auszugehen, dass sämtliche dieser Druckkessel mittels branchenüblicher Schweißverfahren hergestellt wurden.

Der vorliegenden Erfindung liegt die Aufgabe zugrunde, einen Dampfsterilisator anzugeben, der einfacher und kostengünstiger als die aus dem Stand der Technik bekannten Dampfsterilisatoren herstellbar ist und der dennoch die Vorteile eines Doppelmantel-Dampfsterilisators aufweist.

Diese Aufgabe wird durch einen Dampfsterilisator mit den Merkmalen des Anspruchs 1 gelöst. Ein solcher Dampfsterilisator weist einen Druckkessel und ein Mantelelement auf, das druckdicht mit dem Druckkessel verbunden ist. Zwischen dem Druckkessel und dem Mantelelement ist ein Dampfraum ausgebildet.

Gemäß einem Aspekt der vorliegenden Erfindung sind der Druckkessel und das Mantelelement schweißfrei miteinander verbunden. Das heißt, bei der Verbindung zwischen dem Druckkessel und Mantelelement wird gezielt auf eine Schweißverbindung verzichtet. Dadurch lässt sich der Dampfsterilisator viel einfacher herstellen als die aus dem Stand der Technik bekannten Doppelmantel-Dampfsterilisatoren. Da keine aufwändigen Schweißarbeiten durchgeführt werden müssen, lässt sich der erfindungsgemäße Dampfsterilisator deutlich kostengünstiger herstellen als die aus dem Stand der Technik bekannten Doppelmantel-Dampfsterilisatoren. Eine Kosteneinsparung wird auch dadurch erzielt, dass ein kostenintensives Nacharbeiten nach Abschluss der Schweißarbeiten nicht mehr erforderlich ist. Bei den aus dem Stand der Technik bekannten Dampfsterilisatoren werden mittels Schweißverfahren hergestellte Druckkessel zur Sicherstellung einer hinreichenden Oberflächenqualität und einer dauerhaften Korrosionsbeständigkeit elektropoliert, gebeizt, geschliffen und/oder gestrahlt. Diese Verfahrensschritte können bei der vorliegend beanspruchten Erfindung vollständig entfallen. Außerdem wird das Risiko einer versehentlich fehlerhaft ausgeführten Schweißnaht vollständig vermieden. Dadurch wird das Risiko von Rissen in der Schweißnaht eliminiert, wodurch der Anwenderschutz signifikant erhöht wird. Insgesamt lässt sich so ein Doppelmantel-Dampfsterilisator herstellen, der eine höhere Qualität als die aus dem Stand der Technik bekannten Dampfsterilisatoren aufweist und bei dessen Herstellung weniger Nacharbeit nach den einzelnen Verfahrensschritten erforderlich ist.

Eine Kostenreduzierung bei der Herstellung des erfindungsgemäßen Dampfsterilisators ergibt sich auch dadurch, dass eine aufwändige Röntgenprüfung von Schweißnähten vollständig entfallen kann. Dieser Wegfall der Röntgenprüfung spart nicht nur Zeit durch Entfall eines gesamten Verfahrensschritts. Vielmehr wird so auch eine Fehlerquelle eliminiert, die für eine nicht zufriedenstellende Qualität der aus dem Stand der Technik bekannten Dampfsterilisatoren sorgen kann. Denn nicht immer werden alle Fehlerstellen zuverlässig bei der durchgeführten Röntgenprüfung erkannt.

Darüber hinaus ermöglicht die schweißfreie Verbindung zwischen Druckkessel und Mantelelement eine Modularisierung der Herstellung des Dampfsterilisators. Es können unterschiedliche Kessellängen, Mantellängen und Kesselflansche eingesetzt und miteinander kombiniert werden. Diese Modularisierung führt zu einem geringeren Prüfaufwand, zu weniger Anpassungskonstruktionen und zu einem geringeren Zulassungsaufwand im Vergleich zu herkömmlich konstruierten Doppelmantel-Dampfsterilisatoren.

In einer Ausgestaltung kann der Dampfraum bei Bedarf mit einem Innenraum des Druckkessels in Strömungsverbindung gebracht werden. Auf diese Weise lässt sich Dampf vom Dampfraum in den Druckkessel überführen. Zu diesem Zweck ist typischerweise ein Ventil zwischen dem Dampfraum und dem Druckkessel angeordnet.

In einer Ausgestaltung steht der Dampfraum mit einem ersten Dampferzeuger in Strömungsverbindung oder kann mit diesem ersten Dampferzeuger in Strömungsverbindung gebracht werden. Zugleich steht in dieser Ausgestaltung der Innenraum des Druckkessels mit einem zweiten Dampferzeuger in Strömungsverbindung oder kann mit dem zweiten Dampferzeuger in Strömungsverbindung gebracht werden. Dann lassen sich der Dampfraum und der Innenraum des Druckkessels unabhängig voneinander mit Dampf aus separaten Quellen befüllen.

In einer Ausgestaltung stehen sowohl der Dampfraum als auch der Innenraum des Druckkessels mit ein und demselben Dampferzeuger in Strömungsverbindung oder können mit diesem Dampferzeuger in Strömungsverbindung gebracht werden. Dann ist nur ein einziger Dampferzeuger zum Betrieb des Dampfsterilisators notwendig, wobei dennoch die Möglichkeit einer individuellen Befüllung des Dampfraums und des Innenraums des Druckkessels mit Dampf möglich ist.

In einer Ausgestaltung des Dampfsterilisators ist die Verbindung zwischen dem Druckkessel und dem Mantelelement lösbar ausgestaltet. Durch ein Lösen dieser Verbindung ergeben sich umfangreichere Wartungsmöglichkeiten im Vergleich zu Doppelmantel-Dampfsterilisatoren, bei denen der Druckkessel und das Mantelelement miteinander verschweißt sind. So können bei Bedarf beispielsweise einzelne Elemente des Dampfsterilisators viel einfacher ausgetauscht werden als im Falle von verschweißten Dampfsterilisatoren.

Die schweißfreie Verbindung zwischen dem Druckkessel und Mantelelement bedeutet nicht, dass der Dampfsterilisator gar keine Schweißnähte aufweist. Beispielsweise ist es möglich, dass der Druckkessel für sich genommen eine Schweißnaht aufweist (beispielsweise, weil er aus zu einem Rohr gewalzten Edelstahl besteht, das entlang einer Längsnaht eine Schweißverbindung aufweist). Alternativ oder zusätzlich kann das Mantelelement für sich genommen eine Schweißnaht aufweisen. Entscheidend ist jedoch, dass der Druckkessel und das Mantelelement nicht miteinander verschweißt sind. So ist sichergestellt, dass alle vorhandenen Schweißnähte beidseitig kontrolliert werden können. Das würde sich bei einem an den Druckkessel angeschweißten Mantelelement nur von außen bewerkstelligen lassen.

In einer Variante sind der Druckkessel und das Mantelelement nicht nur schweißfrei miteinander verbunden, sondern insgesamt schweißfrei ausgeführt. Das reduziert den Herstellungsaufwand des Dampfsterilisators weiter.

In einer weiteren Ausgestaltung ist der Dampfsterilisator insgesamt schweißfrei ausgeführt. So können der Druckkessel und der Mantel unabhängig voneinander beispielsweise als tiefgezogenes Element gefertigt werden. In einer Ausgestaltung weisen der Druckkessel und/oder das Mantelelement eine runde, elliptische oder anderweitig ovale Querschnittsfläche auf. Dabei sind die Größenverhältnisse zwischen dem Druckkessel und den Mantelelement derart ausgewählt, dass das Mantelelement über den Druckkessel bzw. der Druckkessel über das Mantelelement gestülpt oder gelegt werden kann.

Der Abstand zwischen dem Druckkessel und dem Mantelelement bestimmt die Größe des Dampfraums. Hier sind verschiedenste konstruktive Ausgestaltungen denkbar. In einer Variante ist das Mantelelement in einem Abstand von ca. 2 mm bis ca. 100 mm, insbesondere von ca. 3 mm bis ca. 90 mm, insbesondere von ca. 4 mm bis ca. 80 mm, insbesondere von ca. 5 mm bis ca. 70 mm, insbesondere von ca. 6 mm bis ca. 60 m, insbesondere von ca. 7 mm bis ca. 50 mm, insbesondere von ca. 8 mm bis ca. 45 mm, insbesondere von ca. 9 mm bis ca. 40 mm, insbesondere von ca. 10 mm bis ca. 35 mm, insbesondere von ca. 11 mm bis ca. 30 mm, insbesondere von ca. 12 mm bis ca. 25 mm, insbesondere von ca. 15 mm bis ca. 20 mm, angeordnet. Dadurch ergibt sich ein Dampfraum mit einer nutzbaren radialen Breite, die in den vorgenannten Größenbereichen liegt.

In einer Variante ist ein Verbindungselement zwischen dem Druckkessel und dem Mantelelement angeordnet. Dieses Verbindungselement dichtet den Dampfraum fluiddicht gegenüber einer Umgebung des Dampfsterilisators ab. Dabei sorgt das Verbindungselement für eine schweißfreie Verbindung zwischen dem Druckkessel und dem Mantelelement. Das Verbindungselement fungiert somit als Ersatz für die gemäß dem Stand der Technik vorgesehenen Schweißnähte. Aufgrund seiner dichtenden Eigenschaften kann das Verbindungselement auch als Dichtelement bezeichnet werden.

In einer Variante ist das Verbindungselement als Elastomerdichtung ausgestaltet. Diese Elastomerdichtung muss - wie auch ein anderes Verbindungselement - den auf sie bei einem Betriebsdruck von 5 bar relativem Überdruck bis 10 mbar absolutem Unterdruck, insbesondere 4,5 bar relativem Überdruck bis 20 mbar absolutem Unterdruck, insbesondere 4,0 bar relativem Überdruck bis 30 mbar absolutem Unterdruck, insbesondere 3,5 bar relativem Überdruck bis 40 mbar absolutem Unterdruck, insbesondere 3,0 bar relativem Überdruck bis 50 mbar absolutem Unterdruck, insbesondere 2,5 bar relativem Überdruck bis 60 mbar absolutem Unterdruck, insbesondere 2,0 bar relativem Überdruck bis 70 mbar absolutem Unterdruck, insbesondere 1,5 bar relativem Überdruck bis 80 mbar absolutem Unterdruck auf sie wirkenden Kräften standhalten. Hierfür gibt es verschiedene Konzepte zur Stabilisierung der Elastomerdichtung bzw. eines anderweitig ausgestalteten Verbindungselements.

Gemäß einer Variante ist das Verbindungselement mit dem Druckkessel und/oder mit dem Mantelelement verklebt. Durch die zusätzliche Verklebung lässt sich eine besonders hohe Festigkeit der Verbindung zwischen dem Verbindungselement auf der einen Seite und dem Druckkessel und/oder dem Mantelelement auf der anderen Seite erreichen. Allerdings erschwert eine Verklebung des Verbindungselements eine spätere Demontage des Dampfsterilisators, was zu berücksichtigen ist, wenn diese Variante des Dampfsterilisators ausgewählt wird. Auf der anderen Seite ermöglicht eine entsprechende Verklebung eine dauerhafte und hochfeste Verbindung zwischen dem Druckkessel und dem Mantelelement, wobei zusätzlich die dichtenden Eigenschaften des Verbindungselements ausgenutzt werden können.

Um eine hochfeste, aber dennoch lösbare Verbindung zwischen dem Druckkessel und dem Mantelelement zu ermöglichen, weist der Dampfsterilisator in einer Variante mindestens ein Spannelement auf. Dieses Spannelement verläuft zumindest abschnittsweise um einen äußeren Umfang des Druckkessels, und zwar auf oder neben dem Verbindungselement. Das Spannelement kann beispielsweise ringförmig oder ringsegmentförmig sein. Ein ringförmiges Spannelement kann beispielsweise als Spannring ausgestaltet sein. Wenn das Spannelement auf dem Verbindungselement verläuft, kann durch das Spannelement eine zusätzliche radiale Kraft auf das Verbindungselement ausgeübt werden, durch die den Kräften, die auf das Verbindungselement durch einen Überdruck im Dampfraum wirken, hervorragend entgegengewirkt werden kann. Das Spannelement sorgt in dieser Variante dafür, dass das Verbindungselement auch bei einem entsprechend hohen Überdruck innerhalb des Dampfraums sicher für eine fluiddichte Verbindung zwischen dem Druckkessel und dem Mantelelement sorgt.

In einer Ausgestaltung ist das Spannelement als Schrumpfelement ausgestaltet. Beispielsweise eignen sich alle Elastomere, die bei der Betriebstemperatur des Dampfsterilisators nur geringe elastische Eigenschaften aufweisen und eine entsprechende Spannung auf den Druckkessel aufbauen können, bei einer höheren Temperatur als der Betriebstemperatur aber elastischer und dehnbarer werden, zur Herstellung eines entsprechenden Schrumpfelements. Das entsprechende Schrumpfelement wird dann in dem elastischeren Zustand des Elastomers um den Druckkessel herumgelegt, damit es bei einer Abkühlung auf den Druckkessel aufgeschrumpft wird.

In einer Ausgestaltung weist das Spannelement zumindest abschnittsweise eine Profilierung auf. Diese Profilierung schränkt eine Beweglichkeit des Verbindungselements entlang einer Längserstreckungsachse des Dampfsterilisators ein. Die Längserstreckungsachse des Dampfsterilisators verläuft typischerweise in Längsrichtung des Druckkessels. Die Profilierung sorgt dafür, dass das Verbindungselement an der gewünschten Position verbleibt und nicht entlang der Längserstreckungsachse (also nach vorne oder nach hinten in Bezug auf den Druckkessel) verrutscht. Die Profilierung kann beispielsweise in Form einer separaten oder in Form einer in das Spannelement integrierten Profilschelle ausgeführt sein. Wenn die Profilierung als separates Bauteil realisiert ist, werden das Verbindungselement, das Profilelement, welches die Profilierung bereitstellt, und das Spannelement in dieser Reihenfolge sandwichartig miteinander angeordnet. Eine von dem Spannelement ausgeübte radiale Kraft wirkt dann sowohl auf das Profilelement als auch auf das darunterliegende Verbindungselement.

In einer Variante sind der Druckkessel und das Mantelelement im Bereich des Verbindungselements miteinander verschraubt. Ein Spannelement kann zusätzlich vorgesehen sein. Dies ist aber nicht unbedingt erforderlich, da bereits über die Verschraubung eine druckdichte Verbindung realisiert wird.

In einer Variante handelt es sich bei dem Verbindungselement um ein separat hergestelltes, werkzeugfallendes Element, insbesondere eine separat hergestellte, werkzeugfallende Elastomerdichtung. In einer anderen Variante ist die Dichtung an den Druckkessel und/oder an das Mantelelement anvulkanisiert.

Das Verbindungselement kann auch alleine durch einen Klebstoff (ohne gesonderte Dichtung) gebildet werden. Dabei eignen sich insbesondere Strukturklebstoffe zur Ausbildung des Verbindungselements. Strukturklebstoffe sind Hochleistungsklebstoffe, die sehr schnelle und dauerhafte Klebungen erzielen. Derartige Strukturklebstoffe können Verarbeitungsprozesse beschleunigen, große Spalten überbrücken und die Korrosionsgefahr sowie die Fertigungskosten reduzieren. Besonders geeignete Strukturklebstoffe sind Silikon-, Epoxid-, Acrylat- und/oder Polyurethan-Klebstoffe. In Bezug auf die Flexibilität sind insbesondere Acrylat- und/oder Polyurethan-Klebstoffe vorteilhaft. Epoxid-Klebstoffe weisen eine hohe thermische Beständigkeit (je nach Typ zwischen 100 °C und 200 °C), eine hohe Feuchtigkeitsbeständigkeit, eine sehr hohe Scherfestigkeit und sehr gute Metallklebeeigenschaften auf. Für Anwendungen mit thermischer Ausdehnung (wie im Falle einer Verklebung des Druckkessels mit dem Mantelelement) ist es sinnvoll, dem Epoxid-Klebstoff mindestens ein elastifizierendes Additiv zuzusetzen. Die für Epoxid-Klebstoffe typische Sprödigkeit und das bei Epoxid-Klebstoffen beobachtete Entstehen und Wachsen von Rissen in der Klebstoffmatrix wird mit solchen Additiven effektiv verhindert. Derartige elastifizierende Additive können aber auch im Fall anderer Klebstoffe eingesetzt werden.

Hybridklebstoffe sind ebenfalls besonders geeignete Klebstoffe zur Herstellung des Verbindungselements. Derartige Hybridklebstoffe können auf der Basis von Sofortklebstoffen und/oder Strukturklebstoffen eingesetzt werden. Hybridklebstoffe bieten häufig eine schnellere Aushärtung auch bei größeren Fügespalten von bis zu 5 mm. Da die Klebstoffe zum Abdichten des Dampfraumes des Dampfsterilisators eingesetzt werden, kommen Sie direkt mit Wasser und/oder Dampf in Kontakt. Daher sind biologisch unbedenkliche Klebstoffe grundsätzlich vorteilhaft. Klebstoffe, die der ISO-Norm 10993 über die biologische Beurteilung von Medizinprodukten entsprechen, sind dabei besonders geeignet.

Die vorgenannten Klebstoffe, insbesondere Strukturklebstoffe oder Hybridklebstoffe, sind auch sehr gut geeignet, ein separates Verbindungselement am Druckkessel und/oder am Mantelelement zu fixieren. In einer Variante ist es daher vorgesehen, das Verbindungselement mit einem Klebstoff, insbesondere einem der vorgenannten Strukturklebstoffe oder Hybridklebstoffe, an dem Druckkessel und/oder an dem Mantelelement festzulegen.

In einer weiteren Ausgestaltung ist vorgesehen, dass ein Klebstoff auf eine Oberfläche des Druckkessels und/oder des Mantelelements aufgetragen wird, wobei ein separates Verbindungselement als Anlagefläche für den Klebstoff dient und somit die Fläche, die der Klebstoff auf dem Druckkessel und/oder dem Mantelelement einnehmen kann, zumindest einseitig begrenzt.

In einer Ausgestaltung weisen der Druckkessel und/oder das Mantelelement einen nichtrostenden austhenitischen Stahl auf oder sind vollständig aus diesem hergestellt. Dabei können unterschiedliche Materialien für den Druckkessel und für das Mantelelement verwendet werden. Der Stahl ist dabei zumindest beständig gegenüber Wasserdampf und vollentsalztem (VE) Wasser. Demgegenüber ist es nicht erforderlich, dass der Stahl beispielsweise gegenüber Chloriden beständig ist. In einer Variante ist der Stahl gerade nicht chloridbeständig ausgestaltet. Derartige Stähle sind kostengünstiger als chloridbeständige Stähle.

Durch die schweißfreie Ausgestaltung der Verbindung zwischen dem Druckkessel und dem Mantelelement ist es - anders als bei den aus dem Stand der Technik bekannten Doppelmantel-Dampfsterilisatoren - nicht mehr erforderlich, schweißfeste Edelstähle einzusetzen. Dadurch steht ein viel breiteres Materialspektrum für die Herstellung des erfindungsgemäßen Dampfsterilisators zur Verfügung. Beispielsweise lassen sich deutlich kostengünstigere Edelstähle als die üblicherweise eingesetzten Edelstähle verwenden. In einer Ausgestaltung weisen der Druckkessel und/oder das Mantelelement einen Edelstahl mit einer geringeren Korrosionsbeständigkeit als ein Edelstahl des Typs 1.4571, V4A oder AISI316 auf. Dabei ist es in einer Ausgestaltung möglich, den Druckkessel und/oder das Mantelelement aus einer Kombination verschiedener Materialien, insbesondere aus einem Edelstahl und einem anderen Material und/oder aus zwei verschiedenen Edelstählen unterschiedlicher Korrosionsbeständigkeit, herzustellen. Beispielsweise kann der Druckkessel aus einem Edelstahl mit einer niedrigen Korrosionsbeständigkeit und das Mantelelement aus einem Edelstahl mit einer höheren Korrosionsbeständigkeit gefertigt werden. Dann kann bei der Herstellung des Mantelelements eine Schweißnaht in das zur Herstellung des Mantelelements verwendete Material eingebracht werden (beispielsweise, um aus einem flächigen Metallstück ein Rohr zu formen). In einer weiteren Variante bestehen der Druckkessel und/oder das Mantelelement vollständig aus einem der vorgenannten Edelstähle mit einer geringeren Korrosionsbeständigkeit. Geeignete Edelstähle mit einer geringeren Korrosionsbeständigkeit sind beispielsweise Edelstähle des Typs 1.4301, V2A und AISI304.

In einer Variante weist das Mantelelement eine kesselartige Gestalt auf. Dabei entspricht die Gestalt des Mantelelements einer Gestalt des Druckkessels. Das heißt, auch der Druckkessel weist eine kesselartige Gestalt auf. Das Mantelelement umgibt den Druckkessel in dieser Variante auf einer Außenseite des Druckkessels zumindest abschnittsweise. Somit weist der Dampfsterilisator in dieser Variante einen Aufbau zweier übereinander angeordneter Kessel auf. Eine derartige kesselartige Gestalt des Mantelelements (wie auch eine entsprechend kesselartige Gestalt des Druckkessels) lässt sich besonders einfach durch ein Tiefziehen des Mantelelements bzw. des Druckkessels erreichen. Alternativ ist es auch möglich, ein Metallteil zu einem Rohr zu walzen und die beiden in Längsrichtung freien Kanten des derart geformten Rohrs mittels einer Schweißnaht zu verbinden. Diese Schweißnaht verläuft dann entlang der Längserstreckungsrichtung des Dampfsterilisators, ist also als Längsschweißnaht ausgeführt. Um aus dem Rohr ein Mantelelement mit einer kesselartigen Gestalt zu formen, kann ein Klöpperboden einseitig an das Rohr angeschweißt werden.

In einer weiteren Ausgestaltung ist zwischen dem Druckkessel und dem Mantelelement ein Stabilisierungselement angeordnet. Dieses Stabilisierungselement übt eine Zug- oder Druckkraft zwischen dem Druckkessel und dem Mantelelement aus. Das Stabilisierungselement kann beispielsweise im Bereich des Klöpperbodens des Mantelelements und des Klöpperbodens des Druckkessels angeordnet sein. Dann sorgt das Stabilisierungselement dafür, dass sich das Mantelelement und der Druckkessel auch bei einem erhöhten Druck innerhalb des Dampfraums nicht voneinander lösen. Somit unterstützt das Stabilisierungselement in dieser Variante eine Verbindung zwischen dem Mantelelement und dem Druckkessel durch das Verbindungselement und kann dabei in einer Ausgestaltung einen Teil der auf das Mantelelement und den Druckkessel ausgeübten Kräfte während des Betriebs des Dampfsterilisators aufnehmen. Das Stabilisierungselement kann beispielsweise in Form eines Zug-Druck-Ankers ausgestaltet sein und sowohl mit dem Mantelelement als auch mit dem Druckkessel verklebt oder verschraubt sein. Andere schweißfreie Verbindungstechniken wie beispielsweise Nieten oder Kombinationen verschiedener schweißfreier Verbindungstechniken können ebenfalls eingesetzt werden.

In einer Ausgestaltung weist das Mantelelement eine rohrförmige Gestalt auf. Dabei umgibt das Mantelelement den Druckkessel auf einer Außenseite des Druckkessels zumindest abschnittsweise. In dieser Ausgestaltungsvariante umgibt das Mantelelement den Druckkessel also nicht wie ein grundsätzlich gleich ausgestaltetes kesseiförmiges Element, sondern ist lediglich an einen ebenfalls rohrförmigen Abschnitt des Druckkessels angeordnet. So ist in dieser Variante insbesondere der Klöpperboden des Druckkessels nicht von dem Mantelelement umgeben. Auf diese Weise lässt sich der Materialeinsatz für das Mantelelement signifikant gegenüber der zuvor erläuterten Variante reduzieren. Allerdings ist typischerweise der Einsatz von einer größeren Menge Isolationsmaterial im Bereich des Klöpperbodens angezeigt, um eine zu starke Abkühlung eines Innenraums des Druckkessels über den Klöpperboden während des Betriebs des Dampfsterilisators zu vermeiden.

In einer alternativen Ausgestaltung weist das Mantelelement ebenfalls eine rohrförmige Gestalt auf. Allerdings ist das Mantelelement nicht wie bei der zuvor erläuterten Ausgestaltung um den Druckkessel herum angeordnet, sondern innerhalb des Druckkessels. Folglich ist der Dampf raum in dieser Variante auch nicht auf einer Außenseite des Druckkessels, sondern auf einer Innenseite des Druckkessels realisiert. Eine derartige Ausgestaltung ist bei einer schweißtechnischen Verbindung zwischen dem Mantelelement und dem Druckkessel aufgrund der Unzugänglichkeit der Verbindungsstellen zwischen dem Mantelelement und dem Druckkessel nicht realisierbar. Durch die Verbindung des Mantelelements mit dem Druckkessel über ein schweißfreies Verbindungselement wie beispielsweise eine Dichtung wird eine derartige Ausgestaltung aber machbar.

In einer Variante weist der Dampfsterilisator einen Dampferzeuger auf, der unterhalb des Druckkessels angeordnet und in Strömungsverbindung mit dem Dampfraum steht. Dabei ist die Strömungsverbindung typischerweise ventilfrei ausgestaltet, sodass der im Dampferzeuger erzeugte Dampf unmittelbar in den Dampfraum strömen kann, während sich im Dampfraum bildendes Kondensat in den Dampferzeuger zurückfließen kann. Eine derartige Kondensatrückführung ist bei einem unterhalb des Druckkessels angeordneten Dampferzeuger somit besonders einfach möglich. Demgegenüber gestaltet sich eine Kondensatrückführung deutlich aufwendiger, wenn der Dampferzeuger im Verhältnis zum Druckkessel anders positioniert ist.

In einer Variante ist ein Abstand zwischen dem Mantelelement und dem Druckkessel auf einer Unterseite des Druckkessels größer als auf einer Oberseite des Druckkessels. Dadurch lässt sich ein unterhalb des Druckkessels vergrößerter Dampfraum oder aber ein in den Dampfraum integrierter Dampferzeuger realisieren. Um einen entsprechend vergrößerten Abstand zwischen dem Mantelelement und dem Druckkessel bereitstellen zu können, können der Druckkessel und das Mantelelement beispielsweise exzentrisch zueinander angeordnet sein. Darüber hinaus ist es auch möglich, dass das Mantelelement einen Querschnitt aufweist, der - bei Verwendung einer Längsachse des Dampfsterilisators als Symmetrieachse - asymmetrisch ausgestaltet ist. Beispielsweise kann das Mantelelement ein auf der Unterseite des Druckkessels angeordnetes Flüssigkeitsbecken umfassen, das zusammen mit einem entsprechenden Heizelement als Dampferzeuger dient. Ein derartiges Flüssigkeitsbecken bzw. eine derartige Ausformung des Mantelelements auf der Unterseite des Druckkessels kann derart ausgebildet sein, dass sie im Querschnitt des Mantelelements spiegelsymmetrisch oder aber gerade nicht spiegelsymmetrisch zu einer senkrecht zur Längserstreckungsachse des Dampfsterilisators verlaufenden Symmetrieachse ausgestaltet ist.

In einer Variante weist das Mantelelement einen zentralen Bereich und zwei Endabschnitte auf. Dabei ist ein Abstand zwischen dem zentralen Bereich und dem Druckkessel größer als ein Abstand zwischen mindestens einem der Endabschnitte und dem Druckkessel. In einer Ausgestaltung dieser Variante ist der Abstand zwischen beiden Endabschnitten und dem Druckkessel gleich groß, unterscheidet sich aber von dem Abstand zwischen dem zentralen Bereich und dem Druckkessel. In einer anderen Ausgestaltung dieser Variante ist der Abstand zwischen dem zentralen Bereich und dem Druckkessel größer als der Abstand zwischen genau einem Endabschnitt und dem Druckkessel. Dann ist der Abstand zwischen dem anderen Endabschnitt und dem Druckkessel noch größer als der Abstand zwischen dem zentralen Bereich des Mantelelements und dem Druckkessel. Mit anderen Worten ausgedrückt, verläuft das Mantelelement dann von seinem ersten Endabschnitt zu seinem zweiten Endabschnitt mit wachsendem Abstand vom Druckkessel.

In einer Variante weist das Mantelelement mindestens einen Endabschnitt auf, der gegenüber einer Längserstreckungsrichtung des Mantelelements abgewinkelt ist. Dieser Endabschnitt kontaktiert zudem das Verbindungselement. Aufgrund der winkligen Anordnung des Endabschnitts des Mantelelements und der Kontaktierung des Verbindungselements lässt sich eine besonders sichere Verbindung zwischen dem Mantelelement und dem Verbindungselement realisieren. So ist beispielsweise ein Formschluss zwischen dem Verbindungselement und dem Mantelelement besonders einfach realisierbar. Dadurch lässt sich die Druckdichtigkeit der Verbindung zwischen Mantelelement und dem Druckkessel besonders einfach und dauerhaft realisieren.

Typischerweise werden Anschlussstutzen, mit denen der Dampfraum und/oder der Druckkessel eines Doppelmantel-Dampfsterilisators kontaktiert werden, an den Mantel bzw. den Druckkessel des Dampfsterilisators geschweißt. In einer Variante des vorliegend beanspruchten Dampfsterilisators ist mindestens ein Anschlussstutzen, mit dem eine Fluidverbindung zum Dampfraum oder zum Innenraum des Druckkessels hergestellt werden kann, hingegen als Schraubstutzen ausgebildet. Ein derartiger Schraubstutzen hat den Vorteil, dass er schweißlos mit dem Mantelelement oder dem Druckkessel verbunden wird. Durch den Einsatz eines derartigen Schraubstutzens ist es somit nicht erforderlich, zum Zwecke des Einbringens eines derartigen Stutzens einen besonders korrosionsfesten oder schweißbeständigen Edelstahl einzusetzen. Vielmehr gestattet es die Ausgestaltung eines Anschlussstutzens als Schraubstutzen, das Mantelelement und/oder den Druckkessel im gesamten Bereich aus einem weniger korrosionsbeständigen und damit kostengünstigeren Edelstahl herzustellen als dies bei den aus dem Stand der Technik bekannten Dampfsterilisatoren der Fall ist. In einer Ausgestaltung sind mehrere oder sämtliche Anschlussstutzen des Dampfsterilisators als Schraubstutzen ausgestaltet. Dann muss überhaupt keine Schweißverbindung mehr mit dem Druckkessel und/oder den Mantelelement erfolgen, um ein Prozessmedium in einen Innenbereich des Dampfsterilisators einzubringen oder aus dem Innenbereich des Dampfsterilisators abzuziehen.

Ein Aspekt der vorliegenden Erfindung betrifft ein Verfahren zur Herstellung eines Dampfsterilisators gemäß den obigen Erläuterungen. Dieses Verfahren wird durch die nachfolgend erläuterten Schritte gekennzeichnet.

Zunächst werden ein Druckkessel und ein Mantelelement derart zueinander angeordnet, dass ein Dampfraum zwischen dem Druckkessel und dem Mantelelement ausgebildet wird.

Anschließend erfolgt ein schweißfreies, fluiddichtes und druckdichtes Verbinden des Druckkessels und des Mantelelements miteinander. Dieses schweißfreie Verbinden kann beispielsweise über das Anordnen und Fixieren eines Verbindungselements sowohl am Druckkessel als auch am Mantelelement erfolgen. Das Verbindungselement wird dabei typischerweise an einem Endbereich eines gemeinsamen Abschnitts des Druckkessels und das Mantelelements angeordnet, an dem der Dampfraum ohne das Verbindungselement in Strömungsverbindung mit der Umgebung des Dampfsterilisators stehen würde. Dann sorgt das Verbindungselement nicht nur für eine feste Verbindung zwischen dem Dampfraum und dem Druckkessel, sondern auch für eine fluiddichte Verbindung zwischen beiden Elementen.

Sämtliche Varianten und Ausgestaltungen des Dampfsterilisators können in beliebiger Weise miteinander kombiniert werden und können entweder einzeln oder in beliebiger Kombination auf das Herstellungsverfahren übertragen werden. In gleicher Weise können sämtliche Varianten und Ausgestaltungen des Herstellungsverfahrens in beliebiger Weise miteinander kombiniert werden und einzeln oder in beliebiger Kombination auf den Dampfsterilisator übertragen werden.

Einzelheiten von Aspekten der vorliegend beanspruchten Erfindung werden nachfolgend anhand von Ausführungsbeispielen und Figuren näher erläutert. Es zeigen:
- Figur 1: einen Längsschnitt durch ein erstes Ausführungsbeispiel eines Doppelmantel-Dampfsterilisators;
- Figuren 2 bis 9: Längsschnitte durch weitere Ausführungsbeispiele eines Doppelmantel-Dampfsterilisators;
- Figur 10A: einen Längsschnitt durch ein weiteres Ausführungsbeispiel eines Doppelmantel-Dampfsterilisators;
- Figur 10B: einen Querschnitt durch eine im Ausführungsbeispiel der Figur 10A als Verbindungselement eingesetzte Dichtung;
- Figur 11A: einen Längsschnitt durch ein weiteres Ausführungsbeispiel eines Doppelmantel-Dampfsterilisators;
- Figur 11B: einen Querschnitt durch eine im Ausführungsbeispiel der Figur 11A als Verbindungselement eingesetzte Dichtung;
- Figur 12: einen Längsschnitt durch ein weiteres Ausführungsbeispiel eines Doppelmantel-Dampfsterilisators;
- Figuren 13 bis 21: Detailansichten verschiedener Verbindungsvarianten zwischen einem Druckkessel und einem Mantelelement eines Doppelmantel-Dampfsterilisators; und
- Figuren 22 bis 25: Längsschnitte durch weitere Ausführungsbeispiele eines Doppelmantel-Dampfsterilisators.

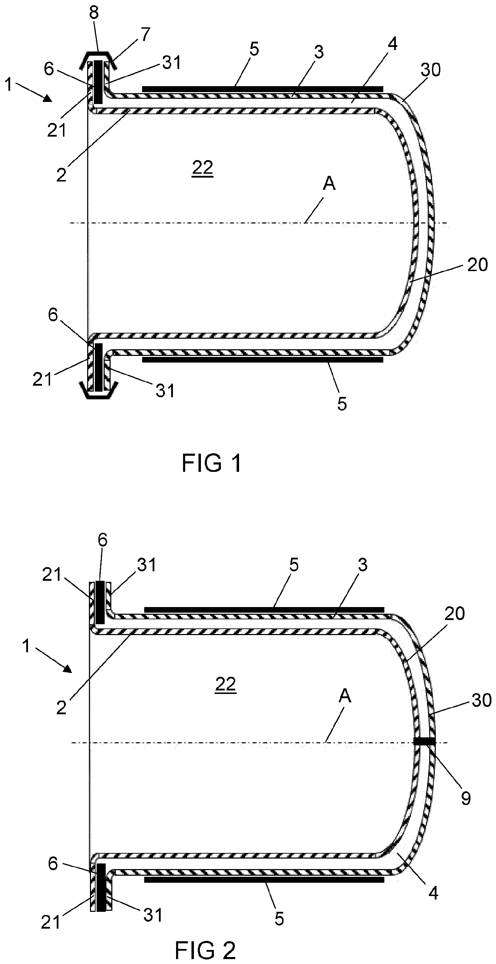

Die Figur 1 zeigt einen Längsschnitt durch ein erstes Ausführungsbeispiel eines Dampfsterilisators 1, der sich entlang einer Längserstreckungsachse A erstreckt. Der Dampfsterilisator 1 weist einen Druckkessel 2 und einen Mantel 3 auf, der als Mantelelement dient. Der Druckkessel 2 ist aus Edelstahl mittels eines Tiefzugverfahrens hergestellt. Erweist eine kesselförmige Gestalt mit einem rückseitigen Klöpperboden 20 auf. An seiner Vorderseite weist der Druckkessel 2 einen Kesselflansch 21 auf, der als Bestandteil des Druckkessels 2 ebenfalls tiefgezogen ist.

Der Mantel 3 weist ebenso wie der Druckkessel 2 eine kesselartige Gestalt mit einem Klöpperboden 30 auf, ist allerdings mit etwas größeren Dimensionen als der Druckkessel 2 ausgestaltet. Dadurch ist es möglich, den Mantel 3 über den Druckkessel 2 zu stülpen. Dabei verbleibt ein Zwischenraum zwischen einer Außenseite des Druckkessels 2 und einer Innenseite des Mantels 3, der als Dampfraum 4 dient. In diesem Dampfraum 4 kann über einen in der Figur 1 nicht dargestellten Anschlussstutzen Wasser eingefüllt werden. Eine auf der Außenseite des Mantels 3 angeordnete Mantelheizung 5 sorgt dann für ein Erwärmen des Mantels 3 und des Dampfraums 4. Dies hat zur Folge, dass das im Dampfraum 4 befindliche Wasser verdampft. Der Dampf kann anschließend über eine in der Figur 1 ebenfalls nicht dargestellte Strömungsverbindung aus dem Dampfraum 4 in einen Innenraum 22 des Druckkessels 2 geleitet werden und dort zur Dampfsterilisation von Sterilisiergut eingesetzt werden.

Damit der Druckkessel 2 und der Mantel 3 fluiddicht miteinander verbunden sind, ist eine Elastomerdichtung 6, die als Verbindungselement dient, zwischen dem Kesselflansch 21 und einem Mantelflansch 31 angeordnet.

Die Elastomerdichtung 6 sorgt dafür, dass kein Dampf aus dem Dampfraum 4 in eine Umgebung des Dampfsterilisators 1 strömen kann. Um auch eine druckdichte Verbindung zwischen dem Druckkessel 2 und dem Mantel 3 sicherzustellen, ist eine Profilschelle 7 mit einem Spannband 8 auf einer radialen Umfangsseite einer Sandwich-Anordnung aus Kesselflansch 21, Elastomerdichtung 6 und Mantelflansch 31 angeordnet. Das Spannband 8 wird dabei auf herkömmlichem Weg so weit gespannt, dass es die vorgenannte Sandwich-Anordnung fest zusammenhält. Dabei wird es von der Profilschelle 7 unterstützt, die eine Seitwärtsbewegung des Kesselflansches 21, der Elastomerdichtung 6 und des Mantelflansches 31 entlang der Längserstreckungsachse A effektiv verhindert. Durch die Profilschelle 7 weist die Anordnung aus dem Spannband 8 und der Profilstelle 7 eine Gestalt wie ein Walmdach auf.

Die Profilschelle 7 kann auch als Profilierung des Spannbandes 8 bezeichnet werden, welches wiederum als ringförmiges Spannelement dient. In dem Ausführungsbeispiel der Figur 1 ist das Spannband 8 vollständig um den äußeren Umfang des Druckkessels 2 gelegt. Grundsätzlich wäre es aber auch möglich, das Spannband 8 nur abschnittsweise um den äußeren Umfang des Druckkessels 2 zu legen. In einem solchen Fall müsste es allerdings an geeigneten Aufnahmevorrichtungen auf der Außenseite des Druckkessels 2 fixiert werden, um seine Spannwirkung auf die Sandwich-Anordnung aus Kesselflansch 21, Elastomerdichtung 6 und Mantelflansch 31 ausüben zu können.

Die Figur 2 zeigt ein weiteres Ausführungsbeispiel eines Dampfsterilisators 1, das dem Ausführungsbeispiel der Figur 1 recht ähnlich ist. In dieser und in allen folgenden Figuren werden vergleichbare Elemente mit denselben Bezugszeichen versehen. Dabei wird bei der Erläuterung der verschiedenen Ausführungsbeispiele insbesondere auf die Unterschiede zwischen den einzelnen Ausführungsbeispielen, nicht jedoch auf deren Gemeinsamkeiten eingegangen.

Bei dem in der Figur 2 dargestellten Dampfsterilisator 1 wird eine druckdichte Verbindung zwischen dem Druckkessel 2 und dem Mantel 3 nicht über ein Spannband 8 mit einer Profilschelle 7 realisiert, sondern mittels eines Zug-Druck-Ankers 9, der als Stabilisierungselement dient. Dieser Zug-Druck-Anker 9 ist im Bereich des Klöpperbodens 20 des Druckkessels 2 sowie des vergleichbar ausgestalteten Klöpperbodens 30 des Mantels 3 angeordnet und fest mit dem Klöpperboden 20 des Druckkessels 2 und dem Körperboden 30 des Mantels 3 verbunden. Dadurch lassen sich der Druckkessel 2 und der Mantel 3 nicht mehr auseinanderziehen. Die Elastomerdichtung 6 ist in gleicher Weise wie beim Ausführungsbeispiel der Figur 1 zwischen dem Kesselflansch 21 und dem Mantelflansch 31 angeordnet und sorgt für eine fluiddichte Abdichtung des Dampfraums 4 gegenüber einer Umgebung des Dampfsterilisators 1. Dabei ist es optional vorgesehen, den Kesselflansch 21 und den Mantelflansch 31 durch die Elastomerdichtung 6 hindurch zu verschrauben, um eine stabilere Befestigung des Mantels 3 am Druckkessel 2 zu erreichen. Eine derartige Verschraubung ist aus Vereinfachungsgründen in keiner der Figuren dargestellt.

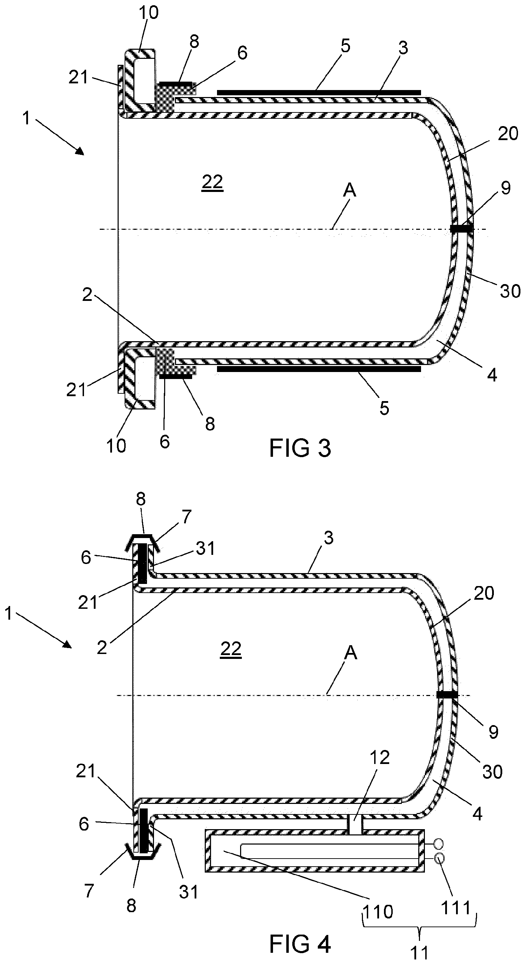

Die Figur 3 zeigt ein weiteres Ausführungsbeispiel eines Dampfsterilisators 1, das dem in der Figur 2 dargestellten Ausführungsbeispiel ähnelt. So ist auch bei dem in der Figur 3 dargestellten Dampfsterilisator 1 ein Zug-Druck-Anker 9 zwischen dem Druckkessel 2 und dem Mantel 3 jeweils im Bereich des Klöpperbodens 20 des Druckkessels 2 sowie des Klöpperbodens 30 des Mantels 3 angeordnet.

Der Mantel 3 weist allerdings auf seiner Vorderseite eine etwas andere Gestalt auf als bei den in den Figuren 1 und 2 gezeigten Ausführungsbeispielen. So ist der Mantel 3 des Dampfsterilisators 1 der Figur 3 ohne Mantelflansch ausgestaltet. Dennoch weist der Mantel 3 eine dem Druckkessel 2 vergleichbare kesseiförmige Gestalt auf. In einem vorderen Endbereich des Mantels 3 ist eine Dichtung 6 angeordnet, die den Dampfraum 4 fluiddicht gegenüber einer Umgebung des Dampfsterilisators abdichtet. Diese Dichtung 6 verläuft dabei auch teilweise auf einer Außenseite des Mantels 3 um die Dichtung 6 druckfest am Druckkessel 2 und am Mantel 3 zu fixieren. Um für eine ebenfalls druckfeste Verbindung zwischen dem Druckkessel 2 und dem Mantel 3 zu sorgen, ist ein Spannband 8 auf einer radialen Außenseite in Umfangsrichtung des Dampfsterilisators 1 um die Dichtung 6 herum gespannt. Mittels dieser Anordnung aus der Dichtung 6 und dem Spannband 8 wird eine druckfeste und fluiddichte Verbindung zwischen dem Druckkessel 2 und dem Mantel 3 gewährleistet, wobei die Druckfestigkeit zusätzlich durch den Zug-Druck-Anker 9 unterstützt wird.

Um eine seitliche Verschiebung der Dichtung 6 entlang der Längserstreckungsachse A des Dampfsterilisators 1 zu verhindern, ist zwischen der Dichtung 6 und dem Kesselflansch 21 zudem ein Kesselkragen 10 angeordnet, der ebenfalls als tiefgezogenes Edelstahlelement ausgestaltet ist. Der Kesselkragen 10 und die durch das Spannband 8 gespannte Dichtung 6 sorgen für eine gegenseitige Lagefixierung auf der Außenseite des Druckkessels 2. Der Kesselkragen 10 sorgt für eine Verstärkung des Kesselflansches 21 und erleichtert somit die Anlenkung einer Tür oder andere Elemente am Druckkessel 2.

Die Figur 4 zeigt ein weiteres Ausführungsbeispiel eines Dampfsterilisators 1, das sehr ähnlich zu den in den Figuren 1 und 2 dargestellten Ausführungsbeispielen ausgestaltet ist. So weist der Dampfsterilisator der Figur 4 sowohl eine Fixierung mittels eines Spannbandes 8 mit einer darunterliegenden Profilschelle 7 im Bereich des Kesselflansches 21 und des Mantelflansches 31 auf als auch zusätzlich eine Verbindung zwischen dem Druckkessel 2 und dem Mantel 3 durch einen Zug-Druck-Anker 9 im Bereich des Klöpperbodens 20 des Druckkessels 2 und des Klöpperbodens 30 des Mantels 3. Dadurch wird eine besonders druckfeste Verbindung zwischen dem Druckkessel 2 und dem Mantel 3 erreicht.

Statt einer Mantelheizung weist der in der Figur 4 dargestellte Dampfsterilisator 1 einen unterhalb des Druckkessels 2 angeordneten Dampferzeuger 11 auf. Dieser Dampferzeuger 11 besteht aus einem Flüssigkeitsbecken 110 und einem in dem Flüssigkeitsbecken 110 angeordneten Rohrheizkörper 111. Der Rohrheizkörper sorgt für ein Verdampfen von Wasser, welches in das Flüssigkeitsbecken 110 eingefüllt ist. Der Dampf steigt dann durch ein Verbindungsrohr 12 zwischen dem Dampferzeuger 11 und dem Dampfraum 4 des Dampfsterilisators 1 in den Dampfraum 4 auf. Dabei ist in dem Verbindungsrohr 12 kein Ventil vorgesehen, sodass im Dampferzeuger 11 erzeugter Dampf hinderungsfrei in den Dampfraum 4 strömen kann. In gleicher Weise kann Kondensat, welches sich im Dampfraum 4 befindet, problemlos durch das Verbindungsrohr 12 zurück in das Flüssigkeitsbecken 110 des Dampferzeugers 11 fließen. Dies ermöglicht einen besonders einfachen und wartungsarmen Betrieb des Dampfsterilisators 1.

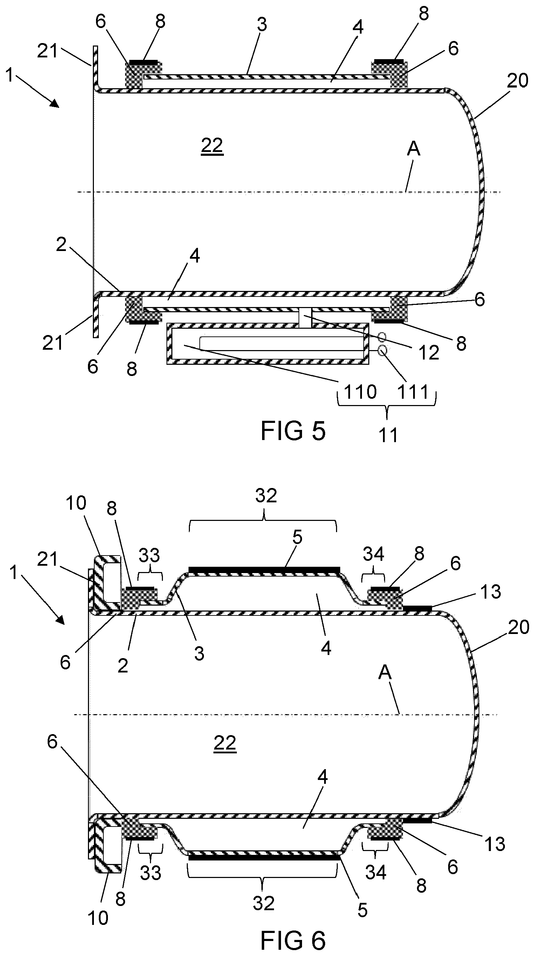

Die Figur 5 zeigt ein weiteres Ausführungsbeispiel eines Dampfsterilisators 1. Im Unterschied zu den bislang erläuterten Ausführungsbeispielen weist der Mantel 3 in diesem Ausführungsbeispiel keine kesselförmige Gestalt auf, sondern ist als zweiseitig offenes Rohr ausgestaltet. Der Dampfraum 4 zwischen dem Druckkessel 2 und dem Mantel 3 fällt somit entsprechend kleiner aus. Dadurch ist insbesondere der Klöpperboden 20 des Druckkessels 2 einschalig ausgestaltet. Dieser als Rohr ausgestaltete Mantel 3 ist indes einfacher herzustellen als der Mantel mit einer kesselförmigen Gestalt wie in den in den Figuren 1 bis 4 gezeigten Ausführungsbeispielen.

Der Mantel 3 wird druck- und fluiddicht mit dem Druckkessel 2 mittels zweier Dichtungen 6 und zweier Spannbänder 8 realisiert. Dabei erstrecken sich die beiden Dichtungen 6 nicht nur in Längserstreckungsrichtung des Dampfsterilisators 1 vor bzw. hinter dem Mantel 3, sondern auch teilweise auf einer Außenseite des Mantels 3. Dadurch wird eine Fixierung der Dichtungen 6 mittels der Spannbänder 8 besonders einfach möglich.

Das Ausführungsbeispiel der Figur 5 ermöglicht eine besonders einfache Wartung des Dampfsterilisators 1. So kann durch Lösen der Spannbänder 8 und Entfernen der Dichtungen 6 der Mantel 3 vom Druckkessel 2 abgezogen und gewartet werden. Bei Bedarf können auch einzelne oder alle Teile des Mantels 3 im Zuge eines derartigen Wartungsprozesses durch neue Teile ausgetauscht werden.

An der Unterseite des Mantels 3 (und damit auch an der Unterseite des Druckkessels 2) ist - wie bei dem in der Figur 4 gezeigten Ausführungsbeispiel - ein Dampferzeuger 11 angeordnet, der mittels eines Verbindungsrohrs 12 mit dem Dampfraum 4 strömungstechnisch verbunden ist. Auch bei dieser Ausgestaltung kann Kondensat aus dem Dampfraum 4 problemlos in den Dampferzeuger 11 zurückfließen, da im Verbindungsrohr 12 kein Ventil vorgesehen ist.

Die Figur 6 zeigt ein weiteres Ausführungsbeispiel eines Dampfsterilisators 1. Auch hier ist der Mantel 3 nicht kesselförmig, sondern rohrartig ausgestaltet. Allerdings weist er nicht - wie der Mantel 3 des in der Figur 5 dargestellten Ausführungsbeispiels - einen über seine gesamte Länge konstanten Querschnitt auf. Vielmehr weist der Mantel in einem zentralen Bereich 32 einen größeren Querschnitt auf als in einem vorderen Endabschnitt 33 und in einem hinteren Endabschnitt 34 des Mantels 3. Dadurch ergibt sich im zentralen Bereich 32 des Mantels 3 ein größerer Abstand zwischen dem Mantel 3 und dem Druckkessel 2 als im vorderen Endabschnitt 33 und im hinteren Endabschnitt 34 des Mantels 3. Folglich weist der Dampfraum 4 ein unterschiedlich großes Volumen in den einzelnen Bereichen des Mantels 3 auf.

Der Mantel 3 ist -wie beim Ausführungsbeispiel der Figur 5 - mittels zweier Dichtungen 6 und um die Dichtungen 6 herum verlaufender Spannbänder 8 am Druckkessel 2 fixiert. Um für eine besonders sichere Fixierung des Mantels 3 am Druckkessel 2 zu sorgen und eine laterale Verschiebung des Mantels 3 entlang der Längserstreckungsachse A entgegenzuwirken, ist hinter der Dichtung 6, die am hinteren Endabschnitt 34 des Mantels 3 angeordnet ist, ein zusätzliches Spannband 13 um den Druckkessel 2 herum geführt. Dieses zusätzliche Spannband 13 verhindert ein Verrutschen des Mantels 3 entlang der Längserstreckungsachse A nach hinten. Außerdem ist im vorderen Bereich des Dampfsterilisators 1 am Kesselflansch 21 abermals ein Kesselkragen 10 angelehnt, der ein Verrutschen der vorderen Dichtung 6, die am vorderen Endabschnitt 33 des Mantels 3 positioniert ist, zum Kesselflansch 21 hin verhindert. Durch diese vordere Dichtung 6 ist zudem der Kesselkragen 10 seinerseits am Kesselflansch 21 festgelegt.

Die gegenüber dem zentralen Bereich 32 mit einem geringeren Querschnitt ausgestalteten Endabschnitte 33 und 34 des Mantels 3 können auch als eingezogene Rohrenden des Mantels 3 bezeichnet werden.

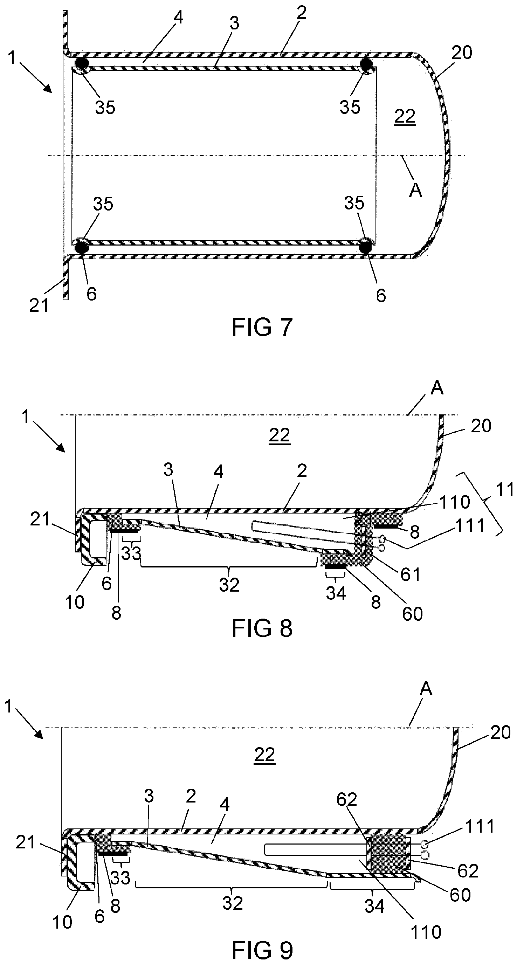

Die Figur 7 zeigt ein weiteres Ausführungsbeispiel eines Dampfsterilisators 1. In diesem Ausführungsbeispiel ist der Mantel 3 nicht auf einer Außenseite des Druckkessels 2 um den Druckkessel 2 herum positioniert, sondern vielmehr in den Innenraum 22 des Druckkessels 2 hineingeschoben. Bei dieser Anordnung ist zwischen dem Mantel 3 dem Druckkessel 2 ein Dampfraum 4 ausgebildet, allerdings zwischen einer Innenseite des Druckkessels 2 und einer Außenseite des Mantels 3.

Für eine druck- und fluiddichte Fixierung des Mantels 3 innerhalb des Druckkessels 2 sorgen dabei zwei Dichtungen 6, von denen eine im vorderen Bereich und die anderen hinteren Bereich des Mantels ringförmig umlaufend auf einer Außenseite des Mantels 3 angeordnet sind. Ein zusätzliches Spannband ist bei dieser Ausgestaltung nicht erforderlich, da ein im Dampfraum 4 oder im Innenraum 22 des Druckkessels 2 aufgebauter Druck für eine sichere Fixierung des Mantels 3 im Druckkessel 2 sorgt. Die Dichtungen 6 sind bei diesem Ausführungsbeispiel wie O-Ringe ausgestaltet und verlaufen in im Mantel 3 ausgebildeten Nuten 35. Dies sorgt für einen sicheren Sitz der Dichtungen 6 an der Außenseite des Mantels 3.

Die Figur 8 zeigt den unteren Bereich eines weiteren Ausführungsbeispiels eines Dampfsterilisators 1. Bei diesem Ausführungsbeispiel ist der Mantel 3 als konisches Rohr ausgestaltet, das konzentrisch um den Druckkessel 2 herum angeordnet ist. Durch die Konizität des Mantels 3 ist der zentrale Bereich 32 des Mantels 3 weiter von der Außenseite des Druckkessels 2 entfernt als der vordere Endabschnitt 33 des Mantels 3. Ferner ist der hintere Endabschnitt 34 noch weiter von der Außenseite des Druckkessels 2 als der zentrale Bereich 32 des Mantels 3 entfernt. Dadurch ergibt sich im hinteren Bereich des Mantels 3 eine signifikante Vergrößerung des Dampfraums 4, sodass dort ein Flüssigkeitsbecken 110 in den Dampfraum 4 integriert ist. Daher weist das in der Figur 8 dargestellte Ausführungsbeispiel auch einen Rohrheizkörper 111 auf, der für eine Verdampfung von Wasser sorgt, welches sich im Flüssigkeitsbecken 110 befindet. Somit weist der Dampfsterilisator 1 der Figur 8 einen integrierten Dampferzeuger 11 auf.

Grundsätzlich könnte der Mantel 3 auch eine konische Ausgestaltung aufweisen, durch die der zentrale Bereich 32 des Mantels 3 weiter von der Außenseite des Druckkessels 2 entfernt ist als der hintere Endabschnitt 34 des Mantels 3. Dann wäre durch die entsprechende Konizität des Mantels 3 der vordere Endabschnitt 33 noch weiter von der Außenseite des Druckkessels 2 als der zentrale Bereich 32 des Mantels 3 entfernt. Diese Variante ist in den Figuren nicht dargestellt.

Aufgrund der Konizität des Mantels 3 ist es erforderlich, dass eine hintere Dichtung 60 anders ausgestaltet ist als die vordere Dichtung 6. So weist die hintere Dichtung 60 einen Stützeinsatz 61 auf, das als radiales Stützelement dient und in Form eines einvulkanisierten Blechs realisiert ist. Die hintere Dichtung 60 ist mittels zweier Spannbänder 8, die in Umfangsrichtung um die hintere Dichtung 60 und den Druckkessel 2 herum verlaufen, fest mit dem Mantel 3 bzw. dem Druckkessel 2 verspannt und sorgt somit für eine relative Verspannung zwischen dem Druckkessel 2 und dem Mantel 3.

Die vordere Dichtung 6 ist in gleicher Weise ausgestaltet wie die Dichtung 6 des in der Figur 6 dargestellten Ausführungsbeispiels. Dabei sorgt abermals ein Kesselkragen 10 für eine Lagefixierung der vorderen Dichtung 6 entlang der Längserstreckungsachse A des Dampfsterilisators 1. Ferner ist die vordere Dichtung 6 mit einem Spannband 8, das radial um die vordere Dichtung 6 und um den Druckkessel 2 herum verläuft, gegenüber dem Druckkessel 2 und dem Mantel 3 verspannt.

Der hintere Bereich des hinteren Endabschnitts 34 des Mantels 3 weist zur besseren Formstabilität zudem eine Abwinkelung gegenüber dem vorderen Bereich des hinteren Endabschnitt 34 auf. Dadurch kann eine besonders sichere Verbindung zwischen dem Mantel 3 und der hinteren Dichtung 60 erreicht werden.

Die Figur 9 zeigt den unteren Bereich eines weiteren Ausführungsbeispiels eines Dampfsterilisators 1, bei dem der Mantel 3 abermals als konzentrisch um den Druckkessel 2 herum angeordnetes konisches Rohr ausgeführt ist. Dabei ist der Mantel 3 im vorderen Bereich wie der Mantel 3 des in der Figur 8 dargestellten Ausführungsbeispiels an dem Druckkessel 2 fixiert. Eine Änderung gibt es lediglich im Bereich der hinteren Dichtung 60, die beim Ausführungsbeispiel der Figur 9 als Ringraumdichtung ausgeführt ist. Diese Ringraumdichtung 60 weist zwei Losflansche 62 auf, die miteinander verschraubt sind. Diese Verschraubung ist aus Gründen der Vereinfachung in der Figur 9 nicht dargestellt. Durch eine Erhöhung der Zugkraft zwischen den beiden Losflanschen 62 wird das Dichtungsmaterial der Ringraumdichtung 60 zusammengequetscht und presst sich gegen den Mantel 3 und den Druckkessel 2. Dadurch wird die Dichtkraft der Ringraumdichtung 60 weiter erhöht. Um ausreichend Platz für die Anordnung der Ringraumdichtung 60 im hinteren Bereich des Mantels 3 zu haben, ist der hintere Endabschnitt 34 des Mantels 3 länger ausgeführt als der vordere Endabschnitt 33. Dadurch ergibt sich auch eine Vergrößerung des Bereichs, in den zur Verdampfung vorgesehene Flüssigkeit in den zum Flüssigkeitsbecken 110 vergrößerten Bereich des Dampfraums 4 eingefüllt werden kann.

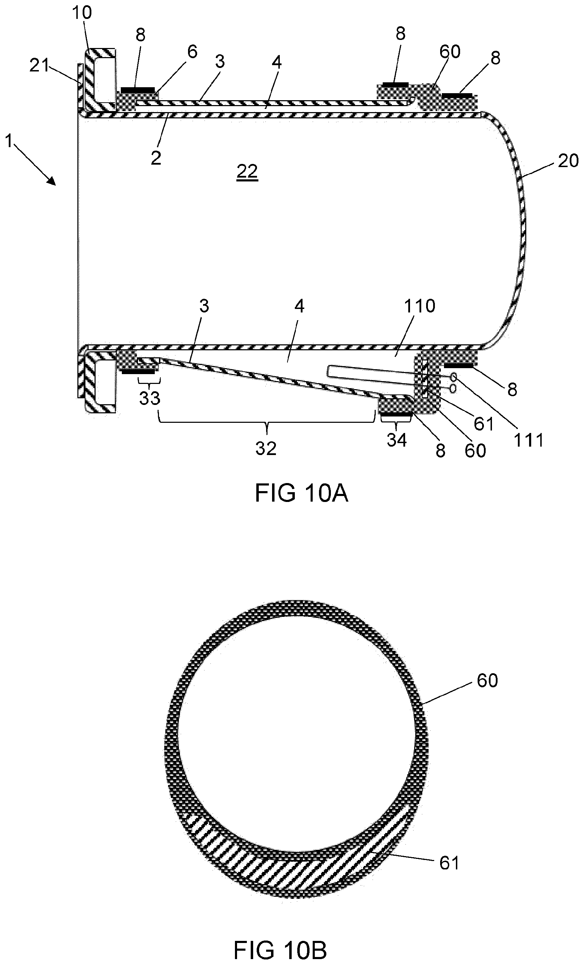

Die Figur 10A zeigt ein weiteres Ausführungsbeispiel eines Dampfsterilisators 1, das dem in der Figur 8 dargestellten Ausführungsbeispiel ähnelt. So handelt es sich bei dem in der Figur 10A dargestellten Dampfsterilisator 1 um einen Dampfsterilisator mit einem runden Druckkessel 2, wobei der Mantel 3 eine asymmetrische Ausgestaltung aufweist. So hat der Mantel 3 auf seiner Unterseite eine konische Form, wie im Zusammenhang mit der Figur 8 bereits erläutert. Auf seiner Oberseite weist der Mantel 3 hingegen einen geraden Verlauf auf, sodass der Dampfraum 4 oberhalb des Druckkessels 2 im gesamten Bereich des Mantels 3 eine konstante Dicke aufweist.

Die mit dem Stützeinsatz 61 versehene hintere Dichtung 60 ist in der Figur 10B gesondert in einer Querschnittsansicht dargestellt. Der Stützeinsatz 61 ist in das Dichtungsmaterial der hinteren Dichtung 60 einvulkanisiert. Wie aus der Darstellung der Figur 10B ersichtlich ist, ist der Stützeinsatz 61 dabei lediglich im unteren Drittel der hinteren Dichtung 60 angeordnet. Mithin ist der Abstand zwischen dem Mantel 3 und dem Druckkessel 2 bereits ab der Hälfte der Höhe der hinteren Dichtung 60 weitgehend konstant; hier weist der Mantel 3 also keinen konischen Verlauf mehr auf und ist insgesamt mit einem geringeren Abstand zum Druckkessel 2 positioniert, sodass es auch keiner gesonderten Abstützung des Mantels 3 über das einvulkanisierte Stützelement 61 der hinteren Dichtung 60 bedarf.

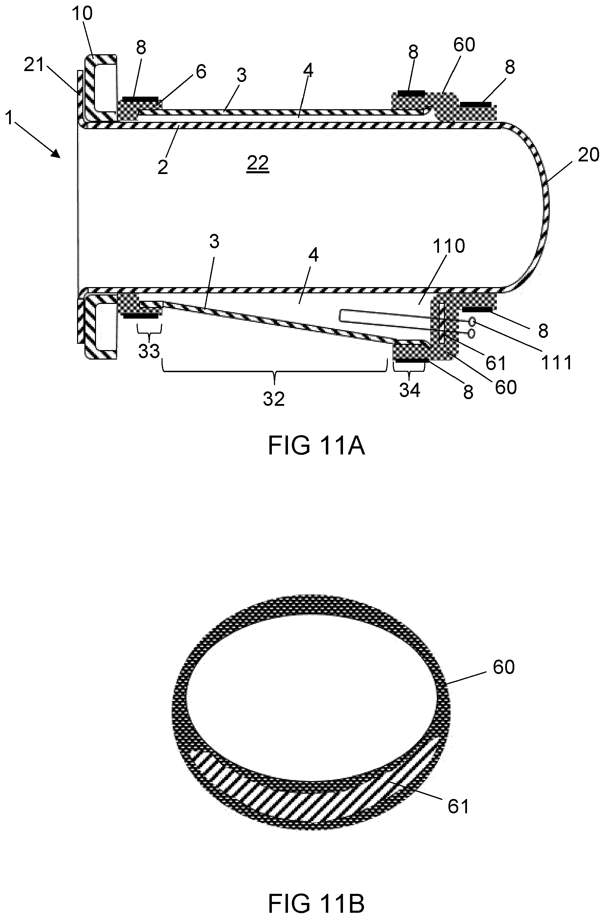

Die Figur 11A zeigt ein weiteres Ausführungsbeispiel eines Dampfsterilisators 1, das dem in der Figur 10A dargestellten Ausführungsbeispiel sehr ähnlich ist. Hier weist der Druckkessel 2 lediglich eine elliptische Form auf, sodass auch der Mantel 3 eine entsprechend elliptische Grundform mit einem konischen Verlauf im unteren Bereich des Mantels 3 zeigt. Dabei ist der Mantel 3 abermals durch das einvulkanisierte Stützelement 61 der hinteren Dichtung 60 im erforderlichen Abstand zum Druckkessel 2 stabilisiert.

Eine Schnittansicht durch die hintere Dichtung 60 ist in der Figur 11B dargestellt. Aus dieser Schnittansicht lässt sich gut die grundsätzliche Form des Druckkessels 2 und des den Druckkessel 2 umgebenden Mantels 3 erkennen.

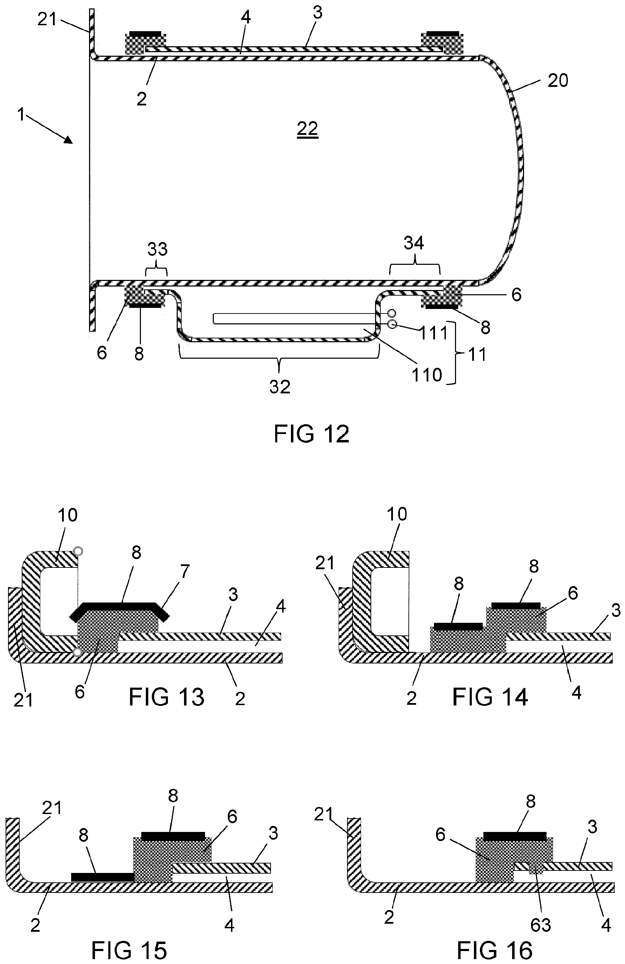

Die Figur 12 zeigt ein weiteres Ausführungsbeispiel eines Dampfsterilisators 1. Dabei weist der Mantel 3 abermals eine rohrförmige Gestalt auf, ist in seinem unteren Bereich aber so geformt, dass er ein Flüssigkeitsbecken 110 bildet, welches zusammen mit einem Rohrheizkörper 111 als integrierter Dampferzeuger 11 dient. Dabei ist das Flüssigkeitsbecken 110 in einem zentralen Abschnitt 32 des Mantels 3 ausgebildet, der von der Außenseite des Druckkessels 2 weiter beabstandet ist als ein vorderer Endabschnitt 33 und ein hinterer Endabschnitt 34 des Mantels 3. Der vordere Endabschnitt 33 und der hintere Endabschnitt 34 des Mantels 3 sind in der bereits oben beschriebenen Art und Weise mittels jeweils einer Dichtung 6 und einem darüber angeordneten Spannband 8 am Druckkessel 2 fixiert.

Die Figuren 13 bis 21 zeigen verschiedene Varianten der druck- und fluiddichten Befestigung des Mantels 3 am Druckkessel 2, die in beliebiger Weise mit sämtlichen der vorstehend erläuterten Ausführungsbeispielen kombiniert werden können, aber hierdurch auch für sich genommen unabhängig von der weiteren konkreten Ausgestaltung des Dampfsterilisators 1 offenbart werden.

In dem in der Figur 13 dargestellten Ausführungsbeispiel ist der Mantel 3 mittels einer Dichtung 6 und einem oberhalb der Dichtung 6 verlaufenden Spannband 8 am Druckkessel 2 fixiert. Zwischen dem Spannband 8 und der Dichtung 6 ist zudem eine Profilschelle 7 angebracht, die für eine besonders gute laterale Festigkeit (entlang der Längserstreckungsrichtung des Dampfsterilisators 1) sorgt. Darüber hinaus ist die Beweglichkeit der Dichtung 6 durch den am Kesselflansch 21 festgelegten Kesselkragen 10 unterbunden.

In der Figur 14 ist zwar auch ein solcher Kesselkragen 10 vorgesehen, allerdings ist er beabstandet zu der Dichtung 6 angeordnet, sodass er nicht unmittelbar eine Lateralverschiebung der Dichtung 6 verhindert. Dennoch ist die Dichtung 6 nicht lateral beweglich, sondern wird mittels zweier Spannbänder 8 gegenüber dem Druckkessel 2 und dem Mantel 3 fixiert, sodass eine zusätzliche Fixierung durch den Kesselkragen 10 nicht erforderlich ist. Die Dichtung 6 weist dabei eine stufenartige Gestalt auf, sodass sich im Längsabschnitt eine Z-förmige Ausgestaltung ergibt.

In dem Ausführungsbeispiel der Figur 15 werden ebenfalls zwei Spannbänder 8 eingesetzt. Allerdings überspannt dabei nur eines dieser Spannbänder 8 die Dichtung 6 und übt damit einen Druck auf den Mantel 3 und den Druckkessel 2 aus. Das zweite Spannband 8 verläuft demgegenüber direkt auf einer Außenseite des Druckkessels 2, lehnt sich mit seiner rückwärtigen Seite aber unmittelbar an die Vorderseite der Dichtung 6 an. Dadurch verhindert dieses zweite Spannband 8 ein Verrutschen der Dichtung 6 nach vorne zum Kesselflansch 21 des Druckkessels 2.

In dem Ausführungsbeispiel der Figur 16 weist die Dichtung 6 eine umlaufende Nase 63 auf, die als Abrutschsicherung für den Mantel 3 dient. Die eigentliche Fixierung der Dichtung 6 erfolgt abermals mittels eines umlaufenden Spannbandes 8, das einen entsprechenden radialen Druck auf die Dichtung 6, den Mantel 3 und den Druckkessel 2 ausübt.

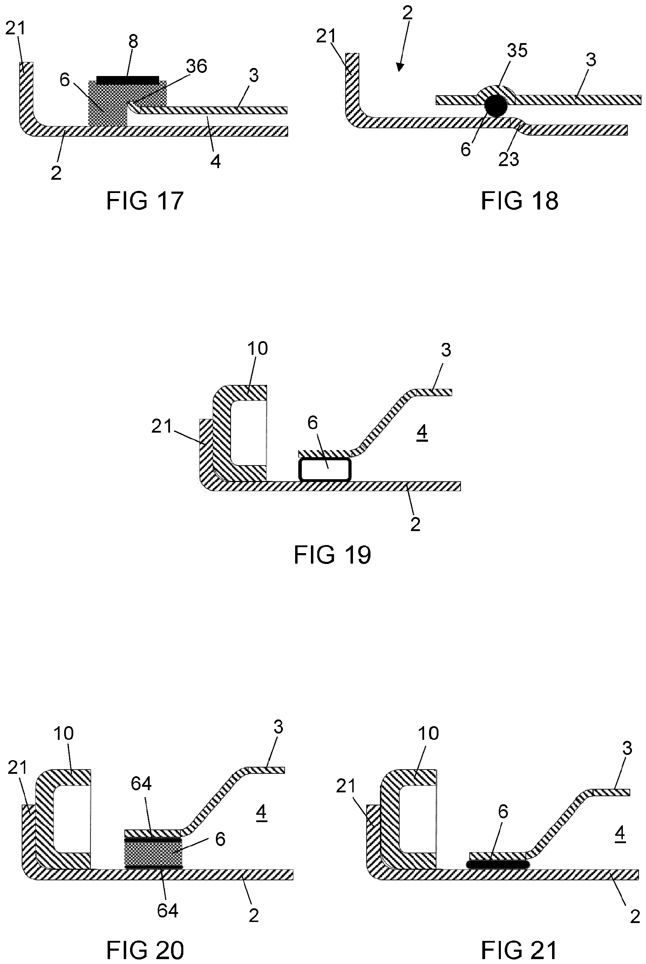

In der Ausgestaltung der Figur 17 weist der Mantel 3 an seinem vorderen Endabschnitt einen gegenüber der Längserstreckungsrichtung des Mantels 3 winklig verlaufenden Abschnitt 36 auf. Dieser winklig verlaufende Abschnitt 36 lehnt sich unmittelbar an die Dichtung 6 an und sorgt somit für einen Formschluss mit der Dichtung 6. Dadurch wird ein besonders sicherer Sitz der Dichtung 6 und des Mantels 3 gegenüber dem Druckkessel 2 erreicht. Die eigentliche Spannkraft zur druck- und fluiddichten Verbindung zwischen dem Mantel 3 und dem Druckkessel 2 wird abermals über ein radial auf der Außenseite der Dichtung 6 verlaufendes Spannband 8 aufgebaut.

In der Figur 18 ist ein Detail der Abdichtung eines im Innenraum 22 des Druckkessels 2 angeordneten Mantels 3 dargestellt. In diesem Zusammenhang wird beispielhaft auch auf das in der Figur 7 dargestellte Ausführungsbeispiel verwiesen. Der Mantel 3 weist in diesem Ausführungsbeispiel eine Nut 35 auf, in der die als O-Ring ausgestaltete Dichtung 6 eingepasst ist. In Anlehnung an eine Rohrpressverbindung kann die Dichtung 6 bei diesem Ausführungsbeispiel während des Montageprozesses mit dem Druckkessel im Bereich der Nut 35 verpresst und normiert werden. So lässt sich die Dichtung 6 in besonders toleranzunempfindlicher Art und Weise in die Nut 35 einbringen. Zudem ist im Druckkessel 2 eine Stufe 23 vorgesehen, die für einen sicheren Sitz des Mantels 3 innerhalb des Druckkessels 2 sorgt.

In der Figur 19 ist ein Ausführungsbeispiel dargestellt, bei dem eine aufblasbare Dichtung 6 als Verbindungselement eingesetzt wird. Diese aufblasbare Dichtung 6 kann mit einem beliebigen Fluid wie etwa Luft aufgeblasen werden. Dadurch lassen sich besonders feine Anpassungen an die Oberflächengeometrie des Druckkessels 2 und des Mantels 3 realisieren. Zudem kann über den Aufblasdruck die Druckfestigkeit und die Fluiddichtigkeit der Verbindung zwischen den Mantel 3 und dem Druckkessel 2 eingestellt werden.

Bei dem in der Figur 20 dargestellten Ausführungsbeispiel weist die Dichtung 6 zwischen dem Mantel 3 und dem Druckkessel 2 zusätzlich zwei Klebstoffschichten 64 auf, mit denen die Dichtung 6 sowohl mit dem Druckkessel 2 als auch mit dem Mantel 3 verklebt ist. Durch eine derartige Verklebung lässt sich eine besonders dichte und dauerhafte Verbindung zwischen der Dichtung 6 und dem Druckkessel 2 sowie zwischen der Dichtung 6 und dem Mantel 3 realisieren, mithin also eine besonders dauerhafte und dichte Verbindung zwischen dem Druckkessel 2 und dem Mantel 3.

In der Figur 21 ist schließlich eine Variante gezeigt, in der das Verbindungselement nicht als Dichtung, sondern als Klebstoffschicht 6 ausgestaltet ist, die den Mantel 3 unmittelbar an den Druckkessel 2 anbindet. Insbesondere beim Einsatz eines Strukturklebstoffs ist das Vorsehen eines zusätzlichen Dichtelements nicht erforderlich, um eine druck- und fluiddichte Verbindung zwischen dem Mantel 3 und dem Druckkessel 2 zu realisieren. Beim Einsatz eines derartigen Klebstoffes ist es auch nicht unbedingt erforderlich, zusätzlich ein Spannband zur Fixierung des Mantels 3 am Druckkessel 2 vorzusehen. Gleichwohl kann ein derartiges Spannband zur zusätzlichen Sicherung der Verbindung zwischen dem Mantel 3 und dem Druckkessel 2 um den Mantel 3 und den Druckkessel 2 herum geführt werden.

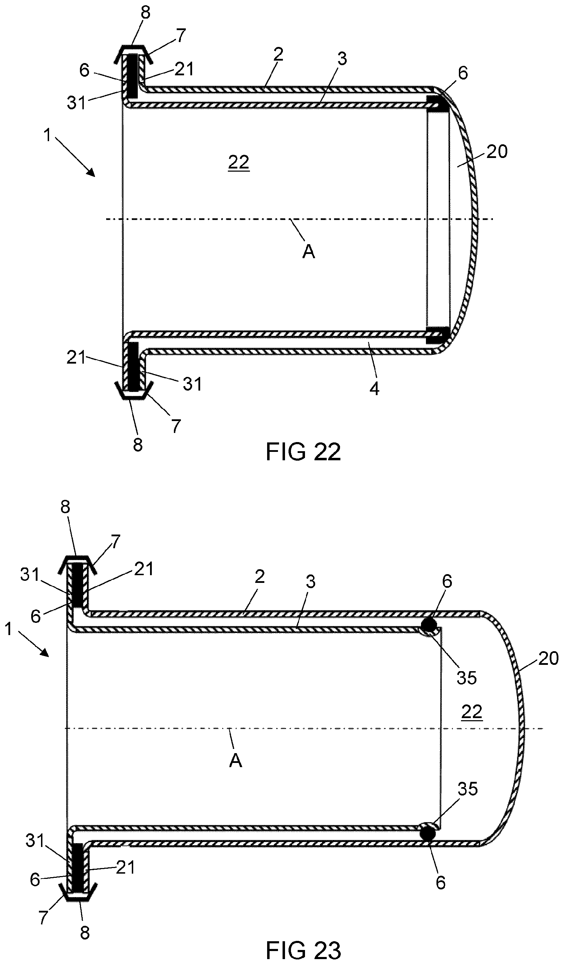

Die Figur 22 zeigt einen Längsschnitt durch ein weiteres Ausführungsbeispiel eines Dampfsterilisators 1. Bei diesem Ausführungsbeispiel ist der Mantel 3 innerhalb des Druckkessels 2 angeordnet, erstreckt sich dabei aber nicht entlang der gesamten Innenfläche des Druckkessels 2. Vielmehr ist der Klöpperboden 20 des Druckkessels 2 einwandig ausgeführt. Dennoch wird ein Dampfraum 4 zwischen dem Mantel 3 und dem Druckkessel 2 ausgebildet. Dies wird dadurch erreicht, dass der Mantel 3 mit einer am Übergang zwischen der zylindrischen Außenfläche des Druckkessels 2 und dem Klöpperboden 20 angeordneten Dichtung 6 mit dem Druckkessel 2 verbunden ist. Dadurch wird der Dampfraum 4 lediglich im vorderen Bereich des Dampfsterilisators 1 gebildet, während eine Außenseite des Klöpperbodens 20 vom Innenraum 22 des Druckkessels 2 nicht durch den Dampfraum 4 getrennt ist.

Bei diesem Ausführungsbeispiel werden - wie auch bei den nachfolgend erläuterten Ausführungsbeispielen - die vorteilhaften Eigenschaften eines Doppelmantelkessels voll ausgenutzt. Dabei bleibt es dennoch möglich, durch einfach ausgeführte Stutzen den Innenraum 22 des Druckkessels 2 mit Dampf zu versorgen oder durch einen ebenfalls einfach ausgeführten Stutzen Druck aus dem Innenraum 22 des Druckkessels 2 abzulassen.

Die Figur 23 zeigt einen Längsschnitt durch ein weiteres Ausführungsbeispiel eines Dampfsterilisators 1. Auch bei diesem Ausführungsbeispiel ist der Mantel 3 innerhalb des Druckkessels 2 angeordnet. Dabei erstreckt er sich nicht über die gesamte Länge des Druckkessels 2 und ist insbesondere - wie beim Ausführungsbeispiel der Figur 22 - nicht im Bereich des Klöpperbodens 20 des Druckkessels 2 ausgebildet.

Die Dichtung 6, die zwischen dem Mantel 3 und dem Druckkessel 2 angeordnet ist, ist bei diesem Ausführungsbeispiel als O-Ring ausgebildet und in eine Nut 35 eingepasst bzw. eingepresst. In dieser Hinsicht wird auch auf das Ausführungsbeispiel der Figur 7 Bezug genommen, das eine ähnliche Ausgestaltung der Dichtung 6 aufweist.

Bei diesem Ausführungsbeispiel ist es besonders einfach möglich, im hinteren Bereich des Druckkessels 2 an dessen tiefster Stelle einen Druckablassstutzen auszubilden, durch den auch Kondensat aus dem Innenraum 22 des Druckkessels 2 abgeführt werden kann. Dabei muss dieser Druckablassstutzen lediglich durch die Wandung des Druckkessels 2 geführt werden, nicht jedoch zusätzlich durch den Dampfraum 4 und die Wandung des Mantels 3.

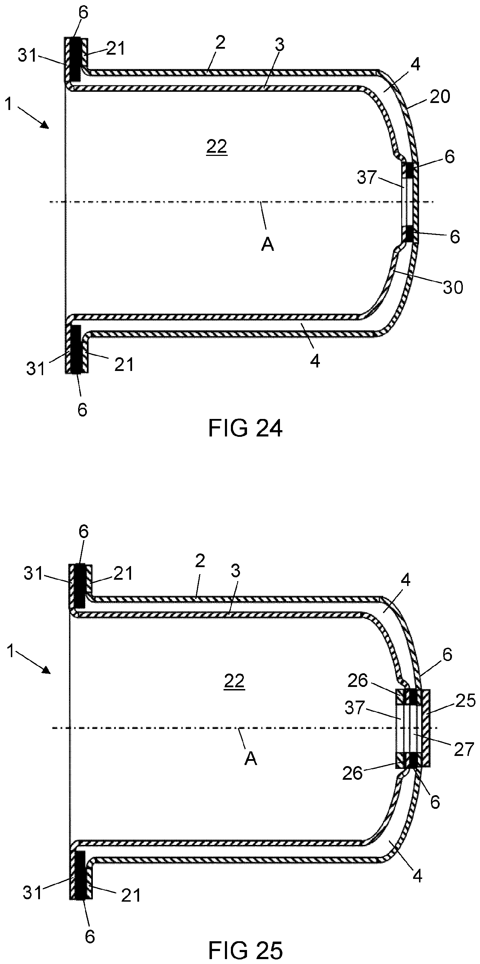

Die Figur 24 zeigt einen Längsschnitt durch ein weiteres Ausführungsbeispiel eines Dampfsterilisators 1, bei dem der Mantel 3 abermals innerhalb des Druckkessels 2 angeordnet ist. Dabei erstreckt sich der Mantel 3 mit einem partiell ausgebildeten Klöpperboden 30 des Mantels 3 entlang des Klöpperbodens 20 des Druckkessels 2. In einem zentralen Bereich rund um die Längserstreckungsachse A herum ist der Klöpperboden 30 des Mantels 3 allerdings unterbrochen und weist eine Öffnung 37 auf. Daher ist in diesem zentralen Bereich nur der Klöpperboden 20 des Druckkessels 2 ausgebildet. Ein oberer Endabschnitt des Klöpperbodens 30 des Mantels 3 ist mit einer Dichtung 6 druck- und fluiddicht mit dem Klöpperboden 20 des Druckkessels 2 verbunden. Gleichermaßen ist ein unterer Endabschnitt des Klöpperbodens 30 des Mantels 3 mit dem Klöpperboden 20 des Druckkessels 2 verbunden. Im Ergebnis wird zwischen dem Mantel 3 und dem Druckkessel 2 abermals ein Dampfraum 4 ausgebildet, der im Bereich des Klöpperbodens 20 des Druckkessels 2 aber nicht durchgehend ist, sondern durch die Öffnung 37 im Klöpperboden 30 des Mantels 3 unterbrochen wird.

Die Figur 25 zeigt schließlich einen Längsschnitt durch ein weiteres Ausführungsbeispiel eines Dampfsterilisators 1, das dem in der Figur 24 dargestellten Ausführungsbeispiel ähnlich ist. Bei dem in der Figur 25 dargestellten Ausführungsbeispiel ist allerdings nicht nur der Klöpperboden 30 des Mantels 3, sondern auch der Körperboden 20 des Druckkessels 2 in einem zentralen Bereich um die Längserstreckungsachse A herum unterbrochen, sodass eine Öffnung 27 im Klöpperboden 20 des Druckkessels 2 gebildet wird. Zusammen mit der Öffnung 37 im Klöpperboden 30 des Mantels 3 würde sich somit eine Verbindung des Innenraums 22 des Druckkessels 2 zu einer Umgebung des Dampfsterilisators 1 ergeben. Damit der Innenraum 22 des Druckkessels 2 dennoch zur Durchführung einer Sterilisationsprozess mit Druck beaufschlagt werden kann, sind die in dem Klöpperboden 30 des Mantels 3 und dem Klöpperboden 20 des Druckkessels 2 ausgebildeten Öffnungen 27, 37 mit einer Flanschplatte 25 verschlossen. Die Flanschplatte 25 verspannt zudem zusammen mit einem im Innenraum 22 des Druckkessels 2 angeordneten Flanschring 26 den Mantel 3, die Dichtung 6 und den Druckkessel 2 im Bereich der in dem Klöpperboden 30 des Mantels 3 und dem Klöpperboden 20 des Druckkessels 2 ausgebildeten Öffnungen 27, 37.

## Patentansprüche

1. Dampfsterilisator, aufweisend einen Druckkessel (2) und ein mit dem Druckkessel (2) druckdicht verbundenes Mantelelement (3), wobei zwischen dem Druckkessel (2) und dem Mantelelement (3) ein Dampfraum (4) ausgebildet ist,
**dadurch gekennzeichnet,**
**dass** der Druckkessel (2) und das Mantelelement (3) schweißfrei miteinander verbunden sind.

2. Dampfsterilisator nach Anspruch 1, **dadurch gekennzeichnet, dass** ein Verbindungselement (6, 60) zwischen dem Druckkessel (2) und dem Mantelelement (3) angeordnet ist, wobei das Verbindungselement (6, 60) den Dampfraum (4) fluiddicht gegenüber einer Umgebung des Dampfsterilisators (1) abdichtet.

3. Dampfsterilisator nach Anspruch 2, **dadurch gekennzeichnet, dass** das Verbindungselement (6, 60) mit dem Druckkessel (2) und/oder mit dem Mantelelement (3) verklebt ist.

4. Dampfsterilisator nach Anspruch 2 oder 3, **dadurch gekennzeichnet, dass** der Dampfsterilisator (1) mindestens ein Spannelement (8) aufweist, das auf oder neben dem Verbindungselement (6, 60) zumindest abschnittsweise um einen äußeren Umfang des Druckkessels (2) verläuft.

5. Dampfsterilisator nach Anspruch 4, **dadurch gekennzeichnet, dass** das Spannelement (8) zumindest abschnittsweise eine Profilierung (7) aufweist, die eine Beweglichkeit des Verbindungselements (6, 60) entlang einer Längserstreckungsachse (A) des Dampfsterilisators (1) begrenzt.

6. Dampfsterilisator nach einem der vorhergehenden Ansprüche, **dadurch gekennzeichnet, dass** der Druckkessel (2) und/oder das Mantelelement (3) einen Edelstahl mit einer geringeren Korrosionsbeständigkeit als Edelstahl des Typs 1.4571, V4A oder AISI316 aufweist.

7. Dampfsterilisator nach einem der vorhergehenden Ansprüche, **dadurch gekennzeichnet, dass** das Mantelelement (3) eine kesselförmige Gestalt aufweist, die einer Gestalt des Druckkessels (2) entspricht, wobei das Mantelelement (3) den Druckkessel (2) auf einer Außenseite des Druckkessels (2) zumindest abschnittsweise umgibt.

8. Dampfsterilisator nach Anspruch 7, **dadurch gekennzeichnet, dass** zwischen dem Druckkessel (2) und dem Mantelelement (3) ein Stabilisierungselement (9) angeordnet ist, das eine Zugkraft zwischen dem Druckkessel (2) und dem Mantelelement (3) ausübt.

9. Dampfsterilisator nach einem der Ansprüche 1 bis 6, **dadurch gekennzeichnet, dass** das Mantelelement (3) eine rohrförmige Gestalt aufweist, wobei das Mantelelement (3) den Druckkessel (2) auf einer Außenseite des Druckkessels (2) zumindest abschnittsweise umgibt.

10. Dampfsterilisator nach einem der Ansprüche 1 bis 6, **dadurch gekennzeichnet, dass** das Mantelelement (3) eine rohrförmige Gestalt aufweist, wobei das Mantelelement (3) innerhalb des Druckkessels (2) angeordnet ist.

11. Dampfsterilisator nach einem der vorhergehenden Ansprüche, **dadurch gekennzeichnet, dass** der Dampfsterilisator (1) einen unterhalb des Druckkessels (2) angeordneten Dampferzeuger (11) aufweist, der in Strömungsverbindung mit dem Dampfraum (4) steht.

12. Dampfsterilisator nach einem der vorhergehenden Ansprüche, **dadurch gekennzeichnet, dass** ein Abstand zwischen dem Mantelelement (3) und dem Druckkessel (2) auf einer Unterseite des Druckkessels (2) größer ist als auf einer Oberseite des Druckkessels (2).

13. Dampfsterilisator nach einem der vorhergehenden Ansprüche, **dadurch gekennzeichnet, dass** das Mantelelement (3) einen zentralen Bereich (32) und zwei Endabschnitte (33, 34) aufweist, wobei ein Abstand zwischen dem zentralen Bereich (32) und dem Druckkessel (2) größer ist als ein Abstand zwischen mindestens einem der Endabschnitte (33, 34) und dem Druckkessel (2).

14. Dampfsterilisator nach einem der vorhergehenden Ansprüche, **dadurch gekennzeichnet, dass** mindestens ein Anschlussstutzen, mit dem eine Fluidverbindung zum Dampfraum (4) oder zum Innenraum (22) des Druckkessels (2) hergestellt werden kann, als Schraubstutzen ausgebildet ist, der schweißlos mit dem Mantelelement (3) oder dem Druckkessel (2) verbunden ist.

15. Verfahren zur Herstellung eines Dampfsterilisators (1) nach einem der vorhergehenden Ansprüche, **gekennzeichnet durch** die folgenden Schritte:
a) Anordnen eines Druckkessels (2) und eines Mantelelements (3) derart, dass ein Dampfraum (4) zwischen dem Druckkessel (2) und dem Mantelelement (3) ausgebildet wird,
b) schweißfreies, fluiddichtes und druckdichtes Verbinden des Druckkessels (2) und des Mantelelements (3) miteinander.
